# FASCICULE DE BREVET EUROPEEN

(11) **EP 2 011 787 B1**
(45) Date de publication et mention de la délivrance du brevet: **01.08.2018**
(21) Numéro de dépôt: 08158622.4
(22) Date de dépôt: 19.06.2008
(51) Int. Cl.: C07D 231/48, C07D 231/54, C07D 231/56, C07D 401/12, C07D 487/04, A61K 8/49, A61Q 5/06

(54) **Composition de coloration comprenant un compose de type azométhinique à motif pyrazolinone**
Färbezusammensetzung enthaltend eine Verbindung vom Azomethin-Typ Verbindung mit einer Pyrazolinon-Einheit
Dying composition comprising an azomethin-type compound with a pyrazolinone moiety

(30) Priorité: 22.06.2007 FR 0755968
(43) Date de publication de la demande: 07.01.2009
(73) Titulaire: L'Oréal, 75008 Paris (FR)
(72) Inventeur: Fadli, Aziz, 77500 Chelles (FR); Metais, Eric, 95320 St Leu la Forêt (FR)
(74) Mandataire: Prevel, Estelle Nicole

(56) Documents cités:
- EP-A- 1 250 909
- EP-A- 1 550 656
- FR-A- 1 488 169
- FR-A- 2 413 660
- US-A- 2 194 201
- DATABASE CA [Online] CHEMICAL ABSTRACTS SERVICE, COLUMBUS, OHIO, US; "Oxidizable anilinoantipyrine derivatives" XP002473683 extrait de STN Database accession no. 1981:497792 & JP 56 039072 A (WAKO PURE CHEMICAL INDUSTRIES, LTD., JAPAN) 14 avril 1981 (1981-04-14)
- EISENSTAEDT EDGAR: "the condensation of aminoantipyrine with aromatic amines" J. ORG. CHEM., vol. 3, 1938, pages 153-163, XP002473677
- JONES PETER ET AL.: "Estimation of phenols by 4-aminoantipyrine method" CAN. J. CHEM., vol. 51, 1973, pages 2860-2868, XP002473678
- KAILASA SURESH KUMAR ET AL.: "Novel reaction for the simple and sensitive spectrophotometric determination of traces of selenium" HELV. CHIM. ACTA, vol. 88, 2005, pages 343-348, XP002473679
- DATABASE BEILSTEIN [Online] BEILSTEIN INSTITUTE FOR ORGANIC CHEMISTRY, FRANKFURT-MAIN, DE; XP002473685 Database accession no. 2802977 -& DATABASE CA [Online] CHEMICAL ABSTRACTS SERVICE, COLUMBUS, OHIO, US; SVOBODOVA, D. ET AL: "Color reaction of phenols with 4-aminoantipyrine. II. Reaction of the phenols substituted in the 2nd and 3rd position" XP002473686 extrait de STN Database accession no. 1970:139485 & COLLECTION OF CZECHOSLOVAK CHEMICAL COMMUNICATIONS , 35(1), 31-44 CODEN: CCCCAK; ISSN: 0010-0765, 1970,
- DATABASE BEILSTEIN [Online] BEILSTEIN INSTITUTE FOR ORGANIC CHEMISTRY, FRANKFURT-MAIN, DE; XP002473688 Database accession no. 5671846 -& DATABASE CA [Online] CHEMICAL ABSTRACTS SERVICE, COLUMBUS, OHIO, US; SAITO, YUTAKA ET AL: "Peroxidase-like catalytic activity of anion-exchange resins modified with metalloporphyrins in the dye formation reaction of N,N-diethylaniline with 4-aminoantipyrine by hydrogen peroxide" XP002505769 extrait de STN Database accession no. 1988:105409 & CHEMICAL & PHARMACEUTICAL BULLETIN, vol. 35, no. 2, 1987, pages 869-872,

## Description

La présente invention a pour objet une composition de coloration des fibres kératiniques, et en particulier des fibres kératiniques humaines telles que les cheveux, comprenant, dans un milieu approprié pour la teinture, au moins un composé de type azométhinique à motif pyrazolinone.

Il est connu de teindre les fibres kératiniques avec des compositions tinctoriales contenant des colorants directs. Ces composés sont des molécules colorées et colorantes ayant une affinité pour les fibres. Il est connu par exemple d'Utiliser des colorants directs du type nitrés benzéniques, des colorants enthraquinoniques, des nitropyridines, des colorants du type azoïque, xanthénique, acridinique, azinique ou triarylméthane.

Habituellement, ces colorants sont appliqués sur les fibres, éventuellement en présence d'un agent oxydant, si l'on souhaite obtenir un effet simultané d'éclaircissement des fibres. Une fois le temps de pose écoulé, les fibres sont rincées, éventuellement lavées et séchées.

Les colorations résultant de l'utilisation de colorants directs sont des colorations souvent chromatiques qui sont cependant temporaires ou semi-permanentes car la nature des interactions qui lient les colorants directs à la fibre kératinique, et leur désorption de la surface et/ou du coeur de la fibre sont responsables de leur faible puissance tinctoriale et de leur relative mauvaise tenue aux lavages ou à la transpiration. Ces colorants directs sont en outre généralement sensibles à la lumière car la résistance du chromophore vis-à-vis des attaques photochimiques est faible, ce qui conduit à un affadissement de la coloration des cheveux dans le temps. La sensibilité de ces colorants à la lumière dépend de leur répartition uniforme ou en agrégats dans et / ou sur la fibre kératinique.

Pour obtenir le même résultat, il est également possible d'utiliser la forme réduite non colorée de ces colorants et de l'appliquer sur les fibres kératiniques en présence d'oxydant pour générer la forme oxydée colorée et colorante.

Le but de la présente invention est de fournir des colorants directs ne présentant pas les inconvénients des colorants directs existants.

En particulier, l'un des buts de la présente invention est de fournir des colorants directs qui permettent d'obtenir une coloration aux nuances variées, puissante, chromatique, esthétique, peu sélective et résistant bien aux diverses agressions que peuvent subir les cheveux tels que les shampooings, la lumière, la sueur et les déformations permanentes, et qui peut s'effacer facilement.

Ce but est atteint avec la présente invention qui a pour objet une composition tinctoriale comprenant, dans un milieu approprié pour la teinture, au moins un composé choisi parmi les composés de type leuco de formule (I), les colorants de type azométhinique à motif pyrazolinone de formule (II) correspondant aux composés de formule (I), leurs formes mésomères, ainsi que leurs sels d'addition avec un acide et leurs solvates : 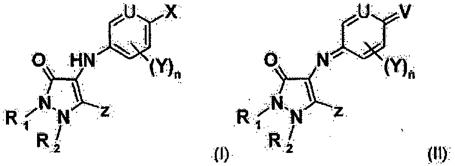 dans laquelle :
- n est un entier compris entre 0 et 3 ;
- U représente CR ou N ;
- R représente
   - un atome d'hydrogène.
   - un radical alkyle en C₁-C₄ éventuellement substitué par un radical hydroxyle ;
   - un radical alcoxy en C₁-C₄ éventuellement substitué par un radical hydroxyle ;
   - un radical (di)alkyl(C₁-C₄)amino dont la partie alkyle est éventuellement substituée par un radical hydroxyle ;
- X représente :
   - un radical hydroxyle;
   - un groupement NR'₁R"₁ avec R'₁ et R"₁ choisis indépendamment parmi un atome d'hydrogène ; un radical alkyle en C₁-C₆ éventuellement substitué par un ou plusieurs radicaux choisis parmi un radical hydroxy, (C₁-C₂₎alcoxy, amino, (di)alkyl(C₁-C₂)amino ; un radical phényle éventuellement substitué par un ou plusieurs radicaux choisis parmi un radical hydroxy, amino, (C₁-C₂)alcoxy;
   lorsque R'₁ et R"₁ sont différents de l'hydrogène, R'₁ et R"₁ peuvent former ensemble avec l'atome d'azote auquel ils sont rattachés un hétérocycle saturé ou insaturé, comportant 5 à 7 chaînons, dont les atomes de carbone peuvent être remplacés par un atome d'oxygène ou d'azote, cet hétérocycle étant éventuellement substitué par un ou plusieurs radicaux choisis parmi un atome d'halogène, un radical amino, (di)alkyl(C₁-C₄)amino, hydroxy, carboxy, carboxamido, (C₁-C₂)alcoxy, alkyle en C₁-C₄ éventuellement substitués par un ou plusieurs radicaux choisis parmi un radical hydroxy, amino, (di)alkylamino, alcoxy, carboxy, sulfonyle ;
   lorsque X représente un groupement NHR'₁ et que U représente un groupement CR dans lequel R désigne un radical alcoxy, alors X et U peuvent former un cycle à 6 chainons de type morpholine, éventuellement substitué par un ou plusieurs groupements alkyles C₁-C₄ ;
- V représente
   ∘ un atome d'oxygène.
   ∘ un groupement NR'₁ avec R'₁ choisi parmi un atome d'hydrogène ; un radical alkyle en C₁-C₈ éventuellement substitué par un ou plusieurs radicaux choisis parmi un radical hydroxy, (C₁-C₂)alcoxy, amino, (di)alkyl(C₁-C₂)amino ; un radical phényle éventuellement substitué par un ou plusieurs radicaux choisis parmi un radical hydroxy, amino, (C₁-C₂)alcoxy
   o un groupement N⁺R'₁R"₁ avec R'₁ et R"₁ choisis indépendamment parmi un atome d'hydrogène; un radical alkyle en C₁-C₈ éventuellement substitué par un ou plusieurs radicaux choisis parmi un radical hydroxy, (C₁-C₂)alcoxy, amino, (di)alkyl(C₁-C₂)amino ; un radical phényle éventuellement substitué par un ou plusieurs radicaux choisis parmi un radical hydroxy, amino, (C₁-C₂)alcoxy,
   lorsque V représente un groupement N⁺R'₁R"₁, l'électroneutralité de la structure (II) est assurée par un anion An-,
   lorsque R'₁ et R"₁ sont différents de l'hydrogène, R'₁ et R"₁ peuvent former ensemble avec l'atome d'azote auquel ils sont rattachés un hétérocycle saturé ou Insaturé, comportant 5 à 7 chaînons, dont les atomes de carbone peuvent être remplacés par un atome d'oxygène ou d'azote, cet hétérocycle étant éventuellement substitué par uh ou plusieurs radicaux Choisis parmi un atome d'halogène, un radical amino, (di)alkyl(C₁-C₄)amino, hydroxy, carboxy, (di)alkylcarboxamido, (C₁-C₂)alcoxy, alkyle en C₁-C₄ éventuellement substitués par un ou plusieurs radicaux choisis parmi un radical hydroxy, amino, (di)alkylamino, alcoxy, carboxy, sulfonyle ;
   lorsque V représente un groupement NR'₁ et que U représente un groupement CR dans lequel R désigne un radical alcoxy, alors V et U peuvent former un cycle à 6 chaînons de type morpholine, éventuellement substitué par un ou plusieurs groupements alkyles C₁-C₄,
- Y, Identiques ou différents, représentent :
   - un radical hydroxy ;
   - un radical alkyle en C₁-C₄ ;
   - un radical hydroxyalkyle C₁-C₄
   - un atome d'halogène tel qu'un atome de chlore, d'iode, de fluor ou de brome ;
   - un atome d'oxygène substitué par un radical choisi parmi un radical alkyle en C₁-C₄, un radical aryle et un radical hétéroaryle, ceux-ci pouvant être substitués par un ou plusieurs radicaux hydroxy ;
   - un groupement NR'₂R'₃ ;
      R'₂ et R'₃, identiques ou différents, peuvent être choisis parmi
      - un atome d'hydrogène ;
      - un radical alkylcarbonyle en C₁-C₄ éventuellement substitué par un groupement ammonium quaternaire tel que par exemple un trialkylammonium ou par un hétérocycle azoté cationique ou non comme par exemple un groupement imidazole, un groupement thiazole, un groupement pyridine, un groupement pipéridine, un groupement pyrrolidine, un groupement pyrimidine, un groupement pyrazine, un groupement imidazolium, un groupement pyridinium, un groupement thiazolium, un groupement pyrrolidinium, un groupement piperidinium, un groupement pyrimidinium, ces hétérocycles azotés étant eux-mêmes éventuellement substitués par un ou plusieurs radicaux alkyle en C₁-C₄ ;
      - un radical aminocarbonyle ;
      - un radical alkyle en C₁-C₆ éventuellement substitué par un ou plusieurs radicaux choisis parmi un radical hydroxy, (C₁-C₂)alcoxy, amino, (di)alkyl(C₁-C₂)amino ;
      - un radical phényle éventuellement substitué par un ou plusieurs radicaux choisis parmi un radical hydroxy, amino, (C₁-C₂)alcoxy ;
      R'₂ et R'₃ peuvent former ensemble avec l'atome d'azote auquel ils sont rattachés un hétérocycle saturé ou insaturé, comportant 5 à 7 chaînons, dont les atomes de carbone peuvent être remplacés par un atome d'oxygène ou d'azote, cet hétérocycle étant éventuellement substitué par un ou plusieurs radicaux choisis parmi un atome d'halogène, un radical amino, (di)alkyl(C₁-C₄)amino, hydroxy, carboxy, (di)alkylcarboxamido, (C₁-C₂)alcoxy, alkyle en C₁-C₄ éventuellement substitués par un ou plusieurs radicaux choisis parmi un radical hydroxy, amino, (di)alkylamino, alcoxy, carboxy, sulfonyle ;
      deux radicaux Y portés par deux atomes de carbone adjacents peuvent former ensemble avec les atomes de carbone auxquels ils sont rattachés un groupement cyclique ou hétérocyclique, saturé ou insaturé, aromatique ou non aromatique, comportant de 5 à 6 chaînons, par exemple un cycle benzène, pyrrole, pyrrolidine, pyrazole, furanne , pyrrolidine, morpholine ou imidazole, éventuellement substitué par un ou plusieurs radicaux alkyle en C₁-C₄ ;
- Z représente :
   - un radical NR₃R₄ ;
   - un radical OR₅ ;
- R₁, R₂, R₃, R₄ et R₅, identiques ou différents, représentent :
   - un radical alkyle en C₁-C₆ éventuellement substitué par un ou plusieurs radicaux choisis parmi un radical OR₅, un radical NR₇R₈, un radical carboxy, un radical carboxylate d'alkyle en C₁-C₄, un radical sulfonique, un radical (di)alkylcarboxamido CONR₇R₈, un radical sulfonamido SO₂NR₇R₈, un radical hétéroaryle à 5 ou 6 chaînons ou phényle éventuellement substitué par un ou plusieurs radicaux choisis parmi un radical (C₁-C₄)alkyle, hydroxy, alcoxy en C₁-C₂, amino, (di)alkyl(C₁-C₂)amino ou hydroxyalkyle en C₁-C₄ ;
   - un radical phenyle éventuellement substitué par un ou plusieurs radicaux choisis parmi un radical (C₁-C₄)alkyle, hydroxy(C₁-C₄)alkyle, hydroxy, alcoxy en C₁-C₂, amino, (di)alkyl(C₁-C₂)amino ;
   - un radical hétéroaryle à 5 ou 6 chaînons éventuellement substitué par un ou plusieurs radicaux choisis parmi un radical (C₁-C₄)alkyle, un radical (C₁-C₂)alcoxy ;
- R₃, R₄ et R₅ peuvent représenter également un atome d'hydrogène ;
- R₆, R₇ et R₈, identiques ou différents, représentent un atome d'hydrogène ; un radical alkyle linéaire ou ramifié en C₁-C₄ éventuellement substitué par un ou plusieurs radicaux choisis parmi un radical hydroxy, un radical alcoxy en C₁-C₂, un radical (di)alkylcarboxamido CONR₉R₁₀, un radical sulfonyle SO₂R₉, un radical phényle éventuellement substitué par un ou plusieurs radicaux choisis parmi un radical (C₁-C₄)alkyle, hydroxy, alcoxy en C₁-C₂, amino, (di)alkyl(C₁-C₂)amino ; un radical phényle éventuellement substitué par un ou plusieurs radicaux choisis parmi un radical (C₁-C₄)alkyle, un radical hydroxy, un radical alcoxy en C₁-C₂, un radical amino, un radical (di)alkyl(C₁-C₂)amino ;
- R₇ et R₈, identiques ou différents, peuvent représenter également un radical (di)alkylcarboxamido CONR₉R₁₀; un radical sulfonyle SO₂R₉ ;
- R₉ et R₁₀, Identiques ou différents, représentent un atome d'hydrogène, un radical alkyle en C₁-C₄ linéaire ou ramifié éventuellement substitués par un ou plusieurs radicaux choisis parmi un radical hydroxy, un radical alcoxy en C₁-C₂ ;
- R₁ et R₂ d'une part, et R₃ et R₄ d'autre part peuvent former ensemble avec les atomes d'azote auxquels ils sont rattachés un hétérocycle saturé ou insaturé, comportant 5 à 7 chaînons, dont les atomes de carbone peuvent être remplacés par un atome d'oxygène ou d'azote, ledit cycle étant éventuellement substitué par un ou plusieurs radicaux choisis parmi un atome d'halogène, un radical amino, (di)alkyl(C₁-C₄)amino, (di)hydroxyalkyl(C₁-C₄)amino, hydroxy, carboxy, (di)alkylcarboxamido, (C₁-C₂)alcoxy, alkyle en C₁-C₄ éventuellement substitué par un ou plusieurs radicaux choisis parmi un radical hydroxy, amino, (di)alkyl(C₁-C₄)amino, alcoxy en C₁-C₂, carboxy, sulfonyle.
- An est un anion ou un mélange d'anions permettant d'assurer l'électroneutralité des structures.

Par sels d'addition, on entend les sels d'acides organiques ou minéraux physiologiquement acceptables des composés de formule (I) et/ou (II).

D'une manière générale, les sels d'addition utilisables sont notamment choisis parmi les sets d'addition avec un acide, tels que l'acide chlorhydrique, l'acide bromhydrique, l'acide sulfurique, l'acide citrique, l'acide succinique, l'acide tartrique, l'acide lactique, l'acide méthanesulfonique, l'acide para-toluènesulfonique, l'acide benzènesulfonique, l'acide phosphorique et l'acide acétique.

La présente Invention a aussi pour objet les nouveaux composés choisis parmi les composés de type leuco de formule (I), les colorants de type azométhinique à motif pyrazolinone de formule (II) correspondant aux composés de formule (I), leurs formes mésomères, leurs sels d'addition avec un acide et leurs solvates.
On connait du document EP1568694 des composés para phénylènediamines N-hétéroarylées dont le substituant R de l'atome d'azote est un hétéroaryle pouvant être un pyrazole différent du groupe pyrazolone présent dans les composés selon l'invention.
On connait également du document EP 1634574 des composés à motifs pyrazolone possédant un atome d'azote basique divalent et non trivalent comme c'est le cas pour les composés selon l'invention, et dont les azotes du motif pyrazolone ne sont pas tous substitués.

La présente invention a également pour objet l'utilisation pour la coloration des fibres kératiniques de composés choisis parmi les composés de type leuco de formule (I), les colorants de type azométhinique à motif pyrazolinone de formule (II) correspondant aux composés de formule (I), leurs formes mésomères, leurs sels d'addition avec un acide et leurs solvates.

La présente invention a également pour objet un procédé pour la coloration des fibres kératiniques mettant en oeuvre dés composés choisis parmi les composés de type leuco de formule (I), les colorants de type azométhinique à motif pyrazolinone de formule (II) correspondant aux composés de formule (I), leurs formes mésomères, leurs sels d'addition avec un acide et leurs solvates.

La présente invention a aussi pour objet un dispositif à plusieurs compartiments pour la mise en oeuvre du procédé conforme à l'invention.

La présénte invention permet en particulier d'obtenir rapidement des colorations chromatiques et résistantes aux diverses agressions que peuvent subir les cheveux, notamment aux shampooings et à la lumière, pouvant être effacées puis reformées tout aussi rapidement

Les composés de type leuco de formule (I), sont incolores ou faiblement colorés et les dérivés azométhiniques à motif pyrazolinone correspondants de formule (II), sont des espèces colorées et colorantes. Il est possible de modifier la structure des composés de formule (I) pour obtenir les composés de formule(II) par ajout d'un agent oxydant, et inversement il est possible de modifier la structure des composés de formule (II) pour obtenir les composés de formule (I) par ajout d'un agent réducteur. Cette modification de structure peut être facilitée par modification du pH et/ou de la température. La formation des composés de formule (I) est ainsi favorisée par un pH acide et/ ou une diminution de la température, la formation des composés de formule (II) est favorisée par un pH basique et/ou une élévation de la température. Un tel comportement permet notamment de modifier facilement la coloration des fibres kératiniques.

A moins d'une indication différente, les bornes des gammes de valeurs qui sont données dans le cadre de l'invention sont incluses dans ces gammes.

Dans le cadre de l'invention, sauf indication contraire, les radicaux alkyle sont linéaires ou ramifiés. Un radical alcoxy est un radical alkyl-O-, le radical alkyle étant tel que défini précédemment.

Un radical (di)alkylamino est un radical amino qui peut être substitué par un ou deux radicaux alkyle.

Un radical (di)alkylcarboxamido est un radical carboxamido qui peut être substitué par un ou deux radicaux alkyle.

Plus particulièrement, dans la formule (I), les radicaux R₁ et R₂, identiques ou différents, sont choisis parmi :
- un radical alkyle en C₁-C₄ éventuellement substitué par un ou plusieurs radicaux choisis parmi un radical hydroxy, un radical (C₁-C₂)alcoxy, un radical amino, un radical (di)alkyl(C₁-C₂)amino ;
- un radical phényle éventuellement substitué par un ou plusieurs radicaux choisis parmi un radical alkyle en C₁-C₄, un radical hydroxyalkyle en C₁-C₄.

De préférence, les radicaux R₁ et R₂, identiques ou non, sont choisis parmi un radical méthyle, éthyle, 2-hydroxyéthyle, 3-hydroxypropyle, 2-hydroxypropyle, phényle.

Encore plus préférentiellement, les radicaux R₁ et R₂ sont identiques et sont choisis parmi un radical méthyle, éthyle, phényle.

Selon un autre mode de réalisation, les radicaux R₁ et R₂ forment ensemble avec les atomes d'azote auxquels ils sont rattachés un cycle à 5 ou 6 chaînons, saturé ou insaturé, éventuellement substitué.

De préférence, les radicaux R₁ et R₂ forment ensemble avec les atomes d'azote auxquels ils sont rattachés un cycle pyrazolidine, pyridazolidine, triazépine éventuellement substitué par un ou plusieurs radicaux choisis parmi un radical alkyle en C₁-C₄, hydroxy, (C₁-C₂)alcoxy, carboxy, (di)alkylcarboxamido, amino, (di)alkyl(C₁-C₂)amino.

De manière encore plus avantageuse, les radicaux R₁ et R₂ forment ensemble avec les atomes d'azote auxquels ils sont rattachés un cycle pyrazolidine, pyridazolidine, triazépine éventuellement substitué par un radical alkyle C₁-C₂.

Selon un mode de réalisation particulier de l'invention, Z représente
- un radical NR₃R₄ dans lequel R₃ et R₄ représentent indépendamment un atome d'hydrogène ou un radical alkyle en C₁-C₄ ou dans lequel R₃ et R₄ forment ensemble avec l'atome d'azote auxquels ils sont rattachés un cycle de 5 à 7 chainons choisi parmi les cycles pyrrolidine, pipéridine, pipérazine.

Selon un mode de réalisation particulier de l'invention, Z représente un radical NR₃R₄.

En ce qui concerne les radicaux R₃, R₄, ces derniers, identiques ou différents, sont plus particulièrement choisis parmi un atome d'hydrogène ; un radical alkyle en C₁-C₄ éventuellement substitué par un ou plusieurs radicaux choisis parmi un radical hydroxy, (C₁-C₂)alcoxy, amino, (di)alkyl(C₁-C₂)amino, carboxy, carboxylate d'alkyle en C₁-C₄ ; un radical phényle éventuellement substitué par un ou plusieurs radicaux choisis parmi un radical hydroxy, amino, (C₁-C₂)alcoxy.

De préférence, les radicaux R₃, R₄, identiques ou non, sont choisis parmi un atome d'hydrogène, un radical méthyle, éthyle, isoprbpyle, 2-hydroxyéthyle, 3-hydroxypropyle, 2-hydroxypropyle, éthyle substitué par un radical carboxylate d'éthyle, éthyle substitué par un radical carboxyle. Selon un mode de réalisation particulier, les radicaux R₃, R₄ et R₅ représentent un atome d'hydrogène ou méthyle.

Selon Un autre mode de réalisation, les radicaux R₃ et R₄ forment ensemble avec l'atome d'azote auquel ils sont rattachés un cycle de 5 à 7 chaînons choisi parmi les hétérocycles pyrrolidine, pipéridine, homopipéridine, pipérazine, homopipérazine, morpholine ; lesdits cycles pouvant être substitués par un ou plusieurs radicaux choisis parmi un radical hydroxy, amino, (di)alkyl(C₁-C₂)amino, (di)hydroxyalkyl(C₁-C₂)amino, (di)alkyl(C₁-C₂)carboxamido, carboxy, alkyle en C₁-C₄ éventuellement substitué par un ou plusieurs radicaux choisis parmi un radical hydroxy, amino, (di)alkylamino en C₁-C₂.

Plus particulièrement, les radicaux R₃ et R₄ forment ensemble avec l'atome d'azote auquel ils sont rattachés un cycle de 5 à 7 chaînons choisi parmi la pyrrolidine, le 2,5-diméthylpyrrolidine, l'acide pyrrolidine-2-carboxylique, l'acide 3-hydroxypyrrolidine-2-carboxylique, l'acide 4-hydroxypyrrolidine-2-carboxylique, la 2,4-dicarboxypyrrolidine, la 3-hydroxy-2-hydroxyméthylpyrrolidine, la 2-carboxamidopyrrolidine, la 3-hydroxy-2-carboxamidopyrrolldine, la 2-(diéthylcarboxamido)pyrrolidine, la 2-hydroxyméthyl pyrrolidine, la 3,4-dihydroxy-2-hydroxyméthyl pyrrolidine, la 3-hydroxypyrrolidine, la 3,4-dihydroxy pyrrolidine, la 3-amino pyrrolidine, la 3-méthylamino pyrrolidine, la 3-diméthylamino-pyrrolidine, la 4-amino-3-hydroxy pyrrolidine, la 3-hydroxy-4-(2-hydroxyéthyl)amino-pyrrolidine, la pipéridine, la 2,6-dimethylpipéridine, la 2-carboxypipéridine, la 2-carboxamidopipéridine, la 2-hydroxyméthylpipéridine, la 3-hydroxy-2-hydroxyméthylpipéridine, la 3-hydroxypipéridine, la 4-hydroxypipéridine, la 3-hydroxyméthylpipéridine, l'homopipéridine, la 2-carboxyhomopipéridine, la 2-carboxamidohomopipéridine, l'homopipérazine, le N-méthyl-homopipérazine, le N-(2-hydroxyéthyl)-homopipérazine, la morpholine.

Dé préférence, les radicaux R₃ et R₄ forment ensemble avec l'atome d'azote auquel ils sont rattachés un cycle de 5 à 7 chaînons choisi parmi la pyrrolidine, la 3-hydroxypyrrolidine, la 3-aminopyrrolidine, la 3-diméthylamino-pyrrolidine, l'acide pyrrolldiné-2-carboxylique, l'acide 3-hydroxypyrrolidine-2-carboxylique, la pipéridine, la 4-hydroxypipéridine, l'homopipéridine, l'homopipérazine, la N-méthyl homopipérazine, la N-(2-hydroxyéthyl)-homopipérazine, la morpholine.

Conformément à un mode de réalisation encore plus préféré de l'invention, les radicaux R₃ et R₄ forment ensemble avec l'atome d'azote auquel ils sont rattachés un cycle à 5 chaînons tel que la pyrrolidine, la 3-hydroxypyrrolidine, la 3-aminopyrrolidine, la 3-diméthylamino-pyrrolidine.

De façon préférée, Z représente un radical amino, un radical éthylamino, un radical isopropylamino, un radical pyrrolidine.

Selon un mode de réalisation particulier de l'invention, U représente CR ou N, et R représente un atome d'hydrogène, un radical alkyle en C₁-C₄, un radical alcoxy en C₁-C₄ éventuellement substitué par un radical hydroxyle, un radical (di)alkyl(C₁-C₄)amino dont la partie alkyle est éventuellement substituée par un radical hydroxyle.

De préférence, U représente CR ou N, et R représente un atome d'hydrogène, un radical méthyle, un radical méthoxy, un radical 2-hydroxyéthoxy, un radical méthylamino, un radical diméthylamino ou hydroxyéthylamino ou un radical dihydroxyéthylamino ou un radical methyl(hydroxyéthyl)amino.

De façon encore plus préférée, U représente CR ou N, et R représente un atome d'hydrogène, un radical méthyle, un radical méthoxy, un radical 2-hydroxyéthoxy.

Selon un mode de réalisation particulier de l'invention, X représente un radical hydroxyle ; un groupement NR'₁R", avec R'₁ et R"₁ choisis indépendamment parmi un atome d'hydrogène ; un radical alkyle en C₁-C₆ éventuellement substitué par un ou plusieurs radicaux hydroxyle. De préférence, X représente un radical hydroxyle; un groupement NR'₁R"₁ avec R'₁ et R", choisis indépendamment parmi un atome d'hydrogène ; un radical méthyle ; un radical 2-hydroxyéthyle.

Selon un autre mode de réalisation, lorsque R'1 et R"1 forment un hétérocycle alors cet hétérocycle peut être choisi parmi les hétérocycles pyrrolidine, pipéridine, homopipéridine, pipérazine, homopipérazine, morpholine ; les dits cycles pouvant être substitués par un ou plusieurs radicaux choisis parmi un atome d'halogène, un radical amino, (di)alkyl(C₁-C₄)amino, hydroxy, carboxy, (di)alkylcarboxamido, (C₁-C₂)alcoxy, alkyle en C₁-C₄ éventuellement substitués par un ou plusieurs radicaux choisis parmi un radical hydroxy, amino, (di)alkylamino, alcoxy, carboxy, sulfonyle . A titre d'exemple, ces hétérocyles sont choisis parmi la pyrrolidine, le 2,5-diméthylpyrrolidine, l'acide pyrrolidine-2-carboxylique, l'acide 3-hydroxypyrrolidine-2-carboxylique, l'acide 4-hydroxypyrrolidine-2-carboxylique, la 2,4-dicarboxypyrrolidine, la 3-hydroxy-2-hydroxyméthylpyrrolidine, la 2-carboxamidopyrrolidine, la 3-hydroxy-2-carboxamidopyrrolidine, la 2-(diéthylcarboxamido)pyrrolidine, la 2-hydroxyméthyl pyrrolidine, la 3,4-dihydroxy-2-hydroxyméthyl pyrrolidine, la 3-hydroxypyrrolidine, la 3,4-dihydroxy pyrrolidine, la 3-amino pyrrolidine, la 3-méthylamino pyrrolidine, la 3-diméthylamino-pyrrolidine, la 4-amino-3-hydroxy pyrrolidine, la 3-hydroxy-4-(2-hydroxyéthyl)amino-pyrrolidine, la pipéridine, la 2,6-diméthylpipéridine, la 2-carboxypipéridine, la 2-carboxamidopipéridine, la 2-hydroxyméthylpipéridine, la 3-hydroxy-2-hydroxyméthylpipéridine, la 3-hydroxypipéridine, la 4-hydroxypipéridine, la 3-hydroxyméthylpipéridine, l'homopipéridine, la 2-carboxyhomopipéridine, la 2-carboxamidohomopipéridine, l'homopipérazine, le N-méthyl-homopipérazine, le N-(2-hydroxyéthyl)-homopipérazine, la morpholine.

Plus préférentiellement, ces hétérocyles sont choisis parmi la pyrrolidine, la 3-hydroxypyrrolidine, la 3-aminopyrrolidine, la 3-diméthylamino-pyrrolidine, l'acide pyrrolidine-2-carboxylique, l'acide 3-hydroxypyrrolidine-2-carboxylique, la pipéridine, la 4-hydroxypipéridine, l'homopipéridine, l'homopipérazine, la N-méthyl homopipérazine, la N-(2-hydroxyéthyl)-homopipérazine, la morpholine.

Conformément à un mode de réalisation encore plus préféré de l'invention, les radicaux R₁ et R"₁ forment ensemble avec l'atome d'azote auquel ils sont rattachés un cycle à 5 chaînons tel que la pyrrolidine, la 3-hydroxypyrrolidine, la 3-aminopyrrolidine, la 3-diméthylamino-pyrrolidine.
Selon un mode particulier de l'invention, V représente
- un atome d'oxygène
- un groupement NR'₁ dans lequel R'1 représente un atome d'hydrogène ou un radical alkyle en C₁-C₆ éventuellement substitué par un ou plusieurs radicaux hydroxyle
- un groupement N+R'1R"1 dans lequel R'1 et R"1 sont choisis indépendamment parmi un atome d'hydrogène, un radical alkyle en C1-C6 éventuellement substitué par un ou plusieurs radicaux hydroxy, ou dans lequel R'1 et R"1 peuvent former ensemble avec l'atome d'azote auxquels ils sont rattachés un hétérocycle saturé ou insaturé, comportant de 5 à 7 chaînons.
De préférence, V représente
- un atome d'oxygène ou
- un groupement NR'₁ dans lequel R'1 représente un atome d'hydrogène ou un radical alkyle en C₁-C₆ éventuellement substitué par un ou plusieurs radicaux hydroxyle,
- un groupement N+R'1R"1 dans lequel R'1 et R"1 sont choisis indépendamment parmi un atome d'hydrogène, un radical alkyle en C1-C6.
De préférence, R'₁ et/ou R"₁ sont choisis parmi un atome d'hydrogène ; un radical méthyle ; un radical 2-hydroxyéthyle.
Selon un autre mode de réalisation particulier de l'invention, X et U, ou respectivement V et U, forment un cycle à 6 chaînons de type morpholine, éventuellement substitué par un ou plusieurs groupements alkyles C₁-C₄.

Selon un autre mode de réalisation particulier de l'invention, Y, identiques ou différents, représentent un radical hydroxy ; un radical alkyle en C₁-C₄ ; un atome d'halogène ; un atome d'oxygène substitué par un radical alkyle en C₁-C₄ pouvant être substitué par un ou plusieurs radicaux hydroxy ; un groupement NR'₂R'₃ ; R'₂ et R'₃, Identiques ou différents, peuvent être choisis parmi un atome d'hydrogène ; un radical alkylcarbonyle en C₁-C₄ éventuellement substitué par par un groupement ammonium quaternaire tel que par exemple un trialkylammonium ou par un hétérocycle azoté cationique ou non comme par exemple un groupement imidazole, un groupement thiazole, un groupement pyridine, un groupement pipéridine, un groupement pyrrolidine, un groupement pyrimidine, un groupement pyrazine, un groupement imidazolium, un groupement pyridinium, un groupement thiazolium, un groupement pyrrolidinium, un groupement piperidinium, un groupement pyrimidinium, ces hétérocycles azotés étant eux-mêmes éventuellement substitués par un ou plusieurs radicaux alkyle en C₁-C₄ ; un radical aminocarbonyle ; un radical alkyle en C₁-C₆ éventuellement substitué par un ou plusieurs radicaux hydroxy ;
R'₂ et R'₃ peuvent former ensemble avec l'atome d'azote auquel ils sont rattachés un hétérocycle saturé comportant de 5 à 7 chaînons ; Dans un mode de réalisation particulier de l'invention, ces hétérocycles sont choisis parmi les hétérocycles pyrrolidine, pipéridine, homopipéridine, pipérazine, homopipérazine, morpholine ; les dits cycles pouvant être substitués par un ou plusieurs radicaux choisis parmi un atome d'halogène, un radical amino, (di)alkyl(C₁-C₄)amino, hydroxy, carboxy, (di)alkylcarboxamido, (C₁-C₂)alcoxy, alkyle en C₁-C₄ éventuellement substitués par un ou plusieurs radicaux choisis parmi un radical hydroxy, amino, (di)alkylamino, alcoxy, carboxy, sulfonyle ;
Deux radicaux Y portés par deux atomes de carbone adjacents peuvent former ensemble avec les atomes de carbone auxquels ils sont rattachés un groupement cyclique ou hétérocyclique, saturé ou insaturé, aromatique ou non aromatique, comportant 5 ou 6 chaînons.

De préférence, Y, identiques ou différents, représentent un radical hydroxy ; un radical méthyle ; un atome de chlore ; un radical méthoxy ; un radical 2-hydroxy éthoxy ; un radical amino ; un radical acétylamino ; un radical (2-hydroxyéthyl)amino ; un radical 2-[(3-méthyl-1H-imidazol-1-ium)acétyl]amino ; un radical pyrrolidin-1-yle ; un radical aminocarbonylamino ; deux radicaux Y portés par deux atomes de carbone adjacents peuvent former ensemble avec les atomes de carbone auxquels ils sont attachés un cycle benzène, pyrrole, pyrrolidine, pyrazole, furanne, morpholine ou imidazole, éventuellement substitué par un ou plusieurs radicaux alkyle en C₁-C₄.

Selon un mode de réalisation particulier, Aₙ est choisi parmi un halogénure tel que chlorure, bromure, fluorure, iodure ; un hydroxyde ; un sulfate ; un hydrogénosulfate ; un phosphate ; un hydrogénophosphate ; un alkyl(C₁-C₆)sulfate tel que par exemple un méthylsulfate ou un éthylsulfate ; un acétate ; un tartrate ; un oxalate ; un alkyl(C₁-C₆)sulfonate tel qu'un méthylsulfonate ; un atylsulfonate substitué ou non substitué par un radical alkyle en C₁-C₄ tel que par exemple un 4-toluylsulfonate ; un méthosulfate ; un perchlorate.

Les composés de type leuco de formule (I), les colorants de type azométhinique à motif pyrazolinone de formule (II) correspondant aux composés de formule (I), peuvent être éventuellement salifiés par des acides minéraux forts tels que par exemple HCl, HBr, HI, H₂SO_{4,} H₃PO₄, ou des acides organiques tels que, par exemple, l'acide acétique, lactique, tartrique, citrique ou succinique, benzènesulfonique, para-toluènesulfonique, formique, méthanesulfonique.

Ils peuvent aussi être sous forme de solvates par exemple un hydrate ou un solvate d'alcool linéaire ou ramifié tel que l'éthanol ou l'isoproponol.

A titre d'exemples de colorants de type azométhinique à motif pyrazolinone de formule (II), on peut citer les composés présentés ci-dessous, An⁻ étant tel que défini précédemment :

| | |
|---|---|
| 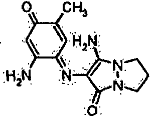 | 3-amino-2-{[(1E)-2-amino-5-methyl-4-oxocyclohexa-2,5-dien-1-ylidene]amino)-6,7-dihydro-1H,5H-pyrazolo[1,2-a]pyrazol-1-one |
| 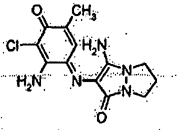 | 3-amino-2-{[(1E)-2-amino-3-chloro-5-methyl-4-oxocyclohexa-2,5-dien-1-ylidene]amino)-6,7-dihydro-1H,5H-pyrazolo[1,2-a]pyrazol-1-one |
| 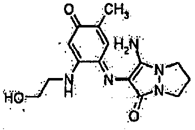 | 3-amino-2-({(1E)-2-[(2-hydroxyethyl)amino)-5-methyl-4-oxocyclohexa-2,5-dien-1-ylidene)amino)-6,1-dihydro-1H,5H-pyrazolo[1,2-a]pyrazol-1-one |
| 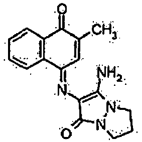 | 3-amino-2-{[(1E)-3-methyl-4-oxonaphthalen-1(4H)-ylidene]amino}-6,7-dihydro-1H,5H-pyrazolo[1,2-a]pyrazol-1-one |
| 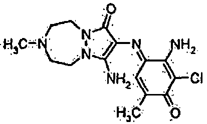 | 9-amino-8-[(2-amino-3-chloro-5-méthyl-4-oxocyclohexa-2,5-dièn-1-ylidène)amino]-3-méthyl-2,3,4,5-tétrahydro-1H,7H-pyrazolo[1,2-a][1,2,5]triazépin-7-one |
| 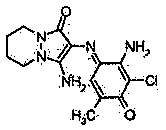 | 3-amino-2-[(2-amino-3-chloro-5-méthyl-4-oxocyclohexa-2,5-dièn-1-ylidène)amino]-5,6,7,8-tétrahydro-1H-pyrazolo[1,2-a]pyridazin-1-one |
| 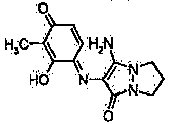 | 3-amino-2-{[(1E)-2-hydroxy-3-methyl-4-oxocyclohexa-2,5-dien-1-ylidene]amino}-6,7-dihydro-1H,5H-pyrazolo[1,2-a]pyrazol-1-one |
| 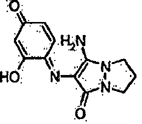 | 3-amino-2-{[(1E)-2-hydroxy-4-oxocyclohexa-2,5-dien-1-ylidene]amino)-6,7-dihydro-1H,5H-pyrazolo-[1,2-a]pyrazol-1-one |
| 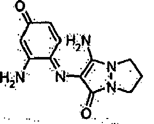 | 3-amino-2-{[(1E)-2-amino-4-oxocyclohexa-2,5-dien-1-ylidene]amino}-6,7-dihydro-1H,5H-pyrazolo[1,2-a]pyrazolo-1-one |
| 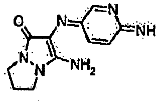 | 3-amino-2-{[(3E)-6-iminopyridin-3(6H)-ylidene]amino}-6,7-dihydro-1H,5H-pyrazolo[1,2-a]pyrazol-1-one |
| 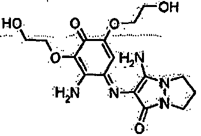 | 3-amino-2-{[(1E)-2-amino-3,5-bis(2-hydroxyethoxy)-4-oxocyclohexa-2,5-dien-1-ylidene]amino}-6,7-dihydro-1H,5H-pyrazolo[1,2-a]pyrazol-1-one |
| 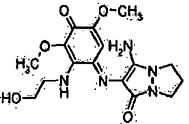 | 3-amino-2-({(1E)-2-[(2-hydroxyethyl}amino]-3,5-dimethoxy-4-oxocyclohexa-2,5-dien-1-ylidene}amino)-6,7-dihydro-1H,5H-pyrazolo[1,2-a]pyrazol-1-one |
| 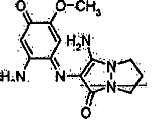 | 3-amino-2-{[(1E)-2-amino-5-methoxy-4-oxocyclohexa-2,5-dien-1-ylidene)amino}-6,7-dihydro-1H,5H-pyrazolo[1,2-a]pyrazol-1-one |
| 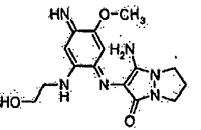 | 3-amino-2-({(1E)-2-[(2-hydroxyethyl)amino]-4-imino-5-méthoxycyclohexa-2,5-dien-1-ylidene}amino)-6,7-dihydro-1H,5H-pyrazolo[1,2-a]pyrazol-1-one |
| 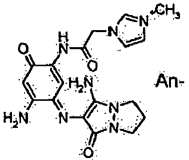 | 1-{2-({(3E)-4-amino-3-[(3-amino-1-oxo-6,7-dihydro-1H,5H-pyrazolo[1,2-a]pyrazol-2-yl)imino)-6-oxocyclohexa-1,4-dien-1-yl}amino)-2-oxoethyl]-3-methyl-1H-imidazol-3-ium; An- |
| 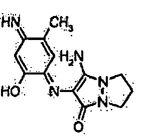 | 3-amino-2-{[(1E)-2-hydroxy-4-imino-5-methylcyclohexe-2,5-dien-1-ylidene]amino}-6,7-dihydro-1H,5H-pyrazolo[1,2-a]pyrazol-1-one |
| 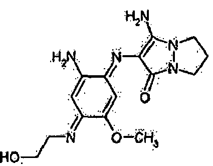 | 3-amino-2-({(1Z-4E)-2-amino-4-[(2-hydroxyethyl)imino]-5-methoxycyclohexa-2,5-dien-1-ylidene)amino)-6,7-dihydro-1H,5H-pyrazolo[1,2-a]pyrazol-1-one |
| 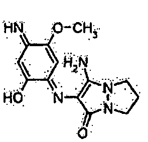 | 3-amino-2-{[(1E)-2-hydroxy-4-imino-5-methoxycyclohexa-2,5-dien-1-ylidene]amino}-6,7-dihydro-1H,5H-pyrazolo[1,2-a]pyrazol-1-one |
| 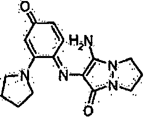 | 3-amino-2-{[(1E)-4-oxo-2-pyrrolidin-1-ylcyclohexa-2,5-dien-1-ylidene]amino}-6,7-dihydro-1H,5H-pyrazolo[1,2-a]pyrazol-1-one |
| 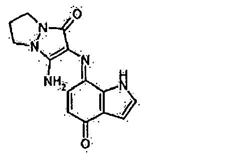 | (7Z)-7-[(3-amino-1-oxo-6,7-dihydro-1H,5H-pyrazolo[1,2-a]pyrazol-2-yl)imino]-1,7-dihydro-4H-indol-4-one |
| 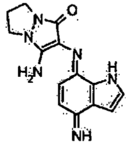 | 3-amino-2-{[(7E)-4-imino-1,4-dihydro-7H-indol-7-ylidene]amino}-6,7-dihydro-1H,5H-pyrazolo[1,2-a]pyrazol-1-one |
| 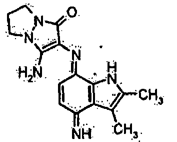 | 3-amino-2-{[(7E)-4-imino-2,3-dimethyl-1,4-dihydro-7H-indol-7-ylidene]amino}-6,7-dihydro-1H, 5H-pyrazolo[1,2-a]pyrazol-1-one |
| 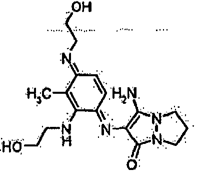 | 3-amino-2-({(1E,4E)-2-[(2-hydroxyethyl)amino}-4-[(2-hydroxyethyl)imino]-3-methylcyclohexa-2,5-dien-1-ylidene)amino)-6,7-dihydro-1H,5H-pyrazolo[1,2-a]pyrazol-1-one |
| 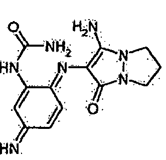 | N-{(6Z)-6-[(3-amino-1-oxo-6,7-dihydro-1H,5H-pyrazolo[1,2-a]pyrazol-2-yl)imino]-3-iminocyclohexa-1,4-dien-1-yl)urea |
| 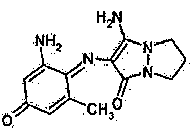 | 3-amino-2-{[(1Z)-2-amino-6-methy)-4-oxocyclohexa-2,5-dien-1-ylidene]amino}-6,7-dihydro-1H,5H-pyrazolo[1,2-a]pyrazol-1-one |
| 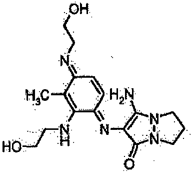 | 3-amino-2-({(1E,4E)-2-[(2-hydroxyethyl)amino]-4-[(2-hydroxyethyl)imino]-3-methylcyclohexa-2,5-dien-1-ylidene}amino)-6,7-dihydro-1H,5H-pyrazolo[1,2-a]pyrazol-1-one |
| 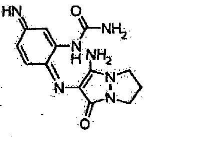 | N-{(6Z)-6-[(3-amino-1oxo-6,7-dihydro-1H,5H-pyrazolo[1,2-a]pyrazol-2-yl)imino]-3-iminocyclohexa-1,4-dien-1-yl)urea |
| 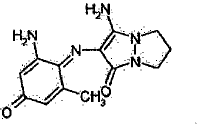 | 3-amino-2-{[(1Z)-2-amino-6-methyl-4-oxocyclohexa-2,5-dien-1-ylidene]amino}-6,7-dihydro-1H,5H-pyrazolo[1,2-a]pyrazol-1-one |
| 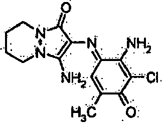 | 3-amino-2-{[(1E)-2-amino-3-chloro-5-methyl-4-oxocyclohexa-2,5-dien-1-ylidene]amino}-5,6,7,8-tetrahydro-1H-pyrazolo[1,2-a]pyridazin-1-one |
| 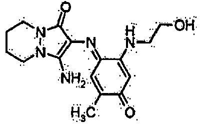 | 3-amino-2-({(1E)-2-[(2-hydroxyethyl)amino]-5-methyl-4-oxocyclohexa-2,5-dien-1-ylidene}amino)-5,6,7,8-tetrahydro-1H-pyrazolo[1,2-a]pyridazin-1-one |
| 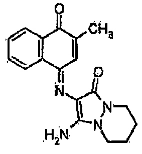 | 3-amino-2-{[(1E)-3-methyl-4-oxonaphthalen-1(4H)-ylidene]amino}-5,6,7,8-totrahydro-1H-pyrazolo[1,2-a]pyridazin-1-one |
| 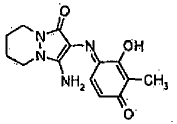 | 3-amino-2-{[(1E)-2-hydroxy-3-methyl-4-oxocyclohexa-2,5-dien-1-ylidene]amino}-5,6,7,8-tetrahydro-1H-pyrazolo[1,2-a]pyridazin-1-one |
| 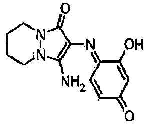 | 3-amino-2-{[(1E)-2-hydroxy-4-oxocyclohexa-2,5-dien-1-ylidene]amino}-5,6,7,8-tetrahydro-1H-pyrazolo[1,2-a]pyridazin-1-one |
| 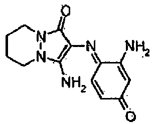 | 3-aminο-2-{[(1E)-2-amino-4-oxocyclohexa-2,5-dien-1-ylidene]amino}-5,6,7,6-tetrahydro-1H-pyrazolo[1,2-a]pyridazin-1-one |
| 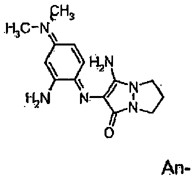 | N-((4E)-3-amino-4-[(3-amino-1-oxo-6,7-dihydro-1H,5H-pyrazolo[1,2-a]pyrazol-2-yl)imino]cyclohexa-2,5-dien-1-ylidene}-N-methylmethanaminium, An- |
| 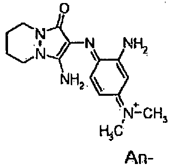 | N-((4E)-3-amino-4-[(3-amino-1-oxo-5,6,7,8-tetrahydro-1H-pyrazolo[1,2-a]pyridazin-2-yl)imino]cyclohexa-2,5-dien-1-ylidene}-N-methylmethanaminium, An- |
| 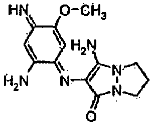 | 3-amino-2-{[(1E)-2-amino-4-imino-5-methoxycyclohexa-2,5-dien-1-ylidene]amino}-6,7-dihydro-1H,5H-pyrazolo[1,2-a]pyrazol-1-one |
| 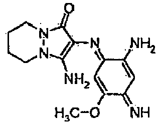 | 3-amino-2-{[(1E)-2-am ino-4-imino-5-methoxycyclohexa-2,5-dien-1-ylidene]amino}-5,6,7,8-tetrahydro-1H-pyrazolo[1,2-a]pyridazin-1-one |
| 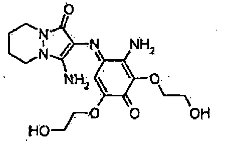 | 3-amino-2-{[(1E)-2-amino-3,5-bis(2-hydroxyethoxy)-4-oxocyclohexa-2,5-dien-1-ylidene]amino}-5,6,7,8-tetrahydro-1H-pyrazolo[1,2-a]pyridazin-1-one |
| 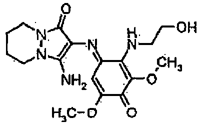 | 3-amino-2-{{(1E)-2-[(2-hydroxyethyl)amino] -3,5-dimethoxy-4-oxocyclohexa-2,5-dien-1-ylidene}amino)-5,6,7,8-tetrahydro-1H-pyrazolo[1,2-a]pyridazin-1-one |
| 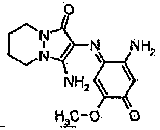 | 3-amino-2-{[(1E)-2-amino-5-methoxy-4-oxocyclohexa-2,5-dien-1-ylidene]amino}-5,6,7,8-tetrahydro-1H-pyrazolo[1,2-a]pyridazin-1-one |
| 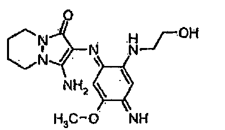 | 3-amino-2-{{(1E)-2-[(2-hydroxyethyl)amino]-4-imino-5-methoxycyclohexa-2,5-dien-1-ylidene}amino)-5,6,7,8-tetrahydro-1H-pyrazolo[1,2-a]pyridazin-1-one |
| 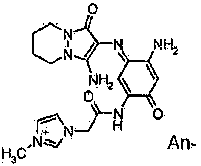 | 1-[2-({(3E)-4-amino-3-[(3-amino-1-oxo-5,6,7,8-tetrahydro-1H-pyrazolo[1,2-a]pyridazin-2-yl)imino]-6-oxocyclohexa-1,4-dien-1-yl}amino)-2-oxoethyl]-3-methyl-1H-imidazol-3-ium, An- |
| 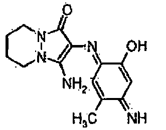 | 3-amino-2-{[(1E)-2-hydroxy-4-imino-5-methylcyclohexa-2,5-dien-1-ylidene]amino}-5,6,7,8-tetrahydro-1H-pyrazolo[1,2-a]pyridazin-1-one |
| 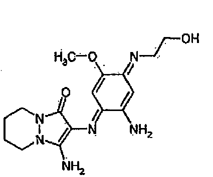 | 3-amino-2-({(1Z,4E)-2-amino-4-[(2-hydroxyethyl)imino]-5-methoxycyclohexa-2,5-dien-1-ylidene}amino)-5,6,7,8-tetrahydro-1H-pyrazolo[1,2-a]pyridazin-1-one |
| 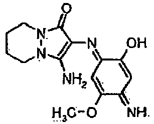 | 3-amino-2-{[(1E)-2-hydroxy-4-imino-5-methoxycyclohexa-2,6-dien-1-ylidene]amino}-5,6,7,8-tetrahydro-1H-pyrazolo[1,2-a]pyridazin-1-one |
| 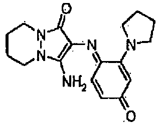 | 3-amino-2-{[(1E)-4-oxo-2-pyrrolidin-1-ylcyclohexe-2,5-dien-1-ylidene]amino}-5,6,7,8-tetrahydro-1H-pyrazolo[1,2-a]pyridazin-1-one |
| 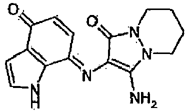 | 3-amino-2-{[(7Z)-4-oxo-1,4-dihydro-7H-indol-7-ylidene]amino}-5,6,7,8-tetrahydro-1H-pyrazolo[1,2-a]pyridozin-1-one |
| 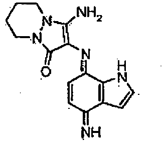 | 3-amino-2-{[(7E)-4-imino-1,4-dihydro-7H-indol-7-ylidene]amino}-5,6,7,8-tetrahydro-1H-pyrazolo[1,2-a]pyridazin-1-one |
| 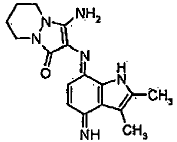 | 3-amino-2-{[(7E)-4-imino-2,3-dimethyl-1,4-dihydro-7H-indol-7-ylidene]amino}-5,6,7,8-tetrahydro-1H-pyrazolo[1,2-a]pyridazin-1-one |
| 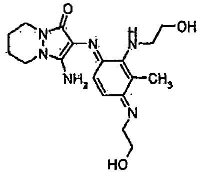 | 3-amino-2-({(1E,4E)-2-[(2-hydroxyethyl)amino]-4-[(2-hydroxyethyl)imino]-3-methylcyclohexa-2,5-dien-1-ylidene}amino)-5,6,7,8-tetrahydro-1H-pyrazolo[1,2-a]pyridazin-1-one |
| 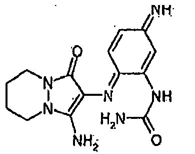 | N-{(6Z)-6-[(3-amino-1-oxo-5,6,7,8-tetrahydro-1H-pyrazolo[1,2-a]pyridazin-2-yl)imino]-3-iminocyclohexa-1,4-dien-1-yl}urea |
| 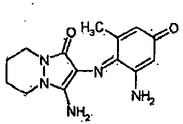 | 3-amino-2-{[(1Z)-2-amino-6-methyl-4-oxocyclohexa-2,5-dien-1-ylidene]amino}-5,6,7,8-tetrahydro-1H-pyrazolo[1,2-a]pyridazin-1-one |
| 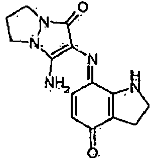 | (7Z)-7-[(3-amino-1-oxo-6,7-dihydro-1H,5H-pyrazolo[1,2-a]pyrazol-2-yl)imino]-1,2,3,7-tetrahydro-4H-indol-4-one |
| 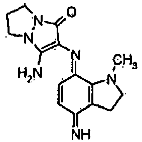 | 3-amino-2-{[(7E)-4-imino-1-methyl-1,2,3,4-tetrahydro-7H-indol-7-ylidene]amino}-6,7-dihydro-1H,5H-pyrazolo[1,2-a]pyrazol-1-one |
| 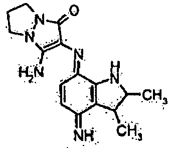 | 3-amino-2-{[(7E)-4-imino-2,3-dimethyl-1,2,3,4-tetrahydro-7H-indol-7-ylidene]amino}-6,7-dihydro-1H,5H-pyrazolo[1,2-a]pyrazol-1-one |
| 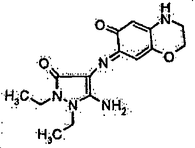 | 7-[(5-amino-1,2-diethyl-3-oxo-2,3-dihydro-1H-pyrazol-4-yl)imino]-3,4-dihydro-2H-1,4-benzoxazin-6(7H)-one |
| 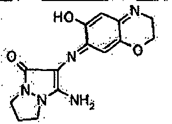 | 3-amino-2-{[(7E)-6-hydroxy-2,3-dihydro-7H-1,4-benzoxazin-7-ylidene]amino}-6,7-dihydro-1H,5H-pyrazolo[1,2-a]pyrazol-1-one |
| 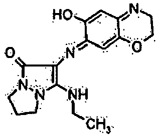 | 3-(ethylamino)-2-{[(7E)-6-hydroxy-2,3-dihydro-7H-1,4-benzoxazin-7-ylidene]amino}-6,7-dihydro-1H,5H-pyrazolo[1,2-a]pyrazol-1-one |
| 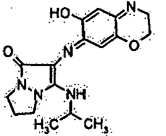 | 2-[(6-hydroxy-2,3-dihydro-7H-1,4-benzoxazin-7-ylidene)amino]-3-(isopropylamino)-6,7-dihydro-1H,5H-pyrazolo[1,2-a]pyrazol-1-one |
| 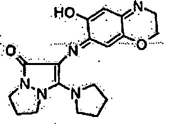 | 2-[(6-hydroxy-2,3-dihydro-7H-1,4-benzoxazin-7-ylidene)amino)-3-pyrrolidin-1-yl-6,7-dihydro-1H,5H-pyrazolo[1,2-a]pyrazol-1-one |
| 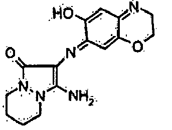 | 3-amino-2-[(6-hydroxy-2,3-dihydro-7H-1,4-benzoxazin-7-ylidène)amino]-5,6,7,8-tetrahydro-1H-pyrazolo[1,2-a]pyridazin-1-one |
| 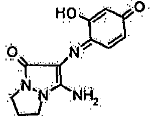 | 3-amino-2-[(2-hydroxy-4-oxocyclohexa-2,5-dien-1-ylidene)amino]-6,7-dihydro-1H,5H-pyrazolo[1,2-a]pyrazol-1-one |
| 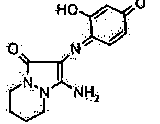 | 3-amino-2-[(2-hydroxy-4-oxocyclohexa-2,5-dien-1-ylidene)amino]-5,6,7,8-tetrahydro-1H-pyraxolo[1,2-a]pyridazin-1-one |
| 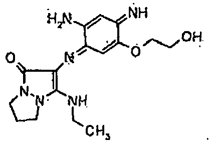 | 2-{[(1E)-2-amino-5-(2-hydroxyethoxy)-4-iminocyclohexa-2,5-dien-1-ylidene]amino}-3-(ethylamino)-6,7-dihydro-1H,5H-pyrazolo[1,2-a]pyrazol-1-one |
| 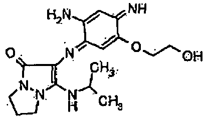 | 2-{[2-amino-5-(2-hydroxyethoxy)-4-iminocyclohexa-2,5-dien-1-ylidene]amino}-3-(isopropylamino)-6,7-dihydro-1H,5H-pyrazolo[1,2-a]pyrazol-1-one |
| 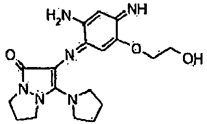 | 2-{[(1E)-2-amino-5-(2-hydroxyethoxy)-4-iminocyclohexa-2,5-dien-1-ylidene]amino}-3-pyrrolidin-1-yl-5,7-dihydro-1H,5H-pyrazolo[1,2-a]pyrazol-1-one |
| 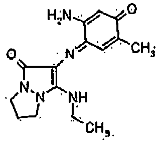 | 2-[(2-amino-5-methyl-4-oxocyclohexa-2,5-dien-1-ylidene)amino]-3-(ethylamino)-6,7-dihydro-1H,5H-pyrazolo[1,2-a]pyrazol-1-one |
| 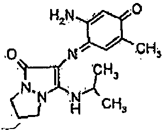 | 2-[(2-amino-5-methyl-4-oxocyclohexa-2,5-dien-1-ylidene)amino]-3-(isopropylamino)-6,7-dihydro-1H,5H-pyrazolo[1,2-a]pyrazol-1-one |
| 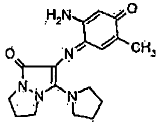 | 2-[(2-amino-5-melhyl-4-oxocyclohexa-2,5-dien-1-ylidene)amino]-3-pyrrolidin-1-yl-6,7-dihydro-1H,5H-pyrazo[1,2-a]pyrazol-1-one |
| 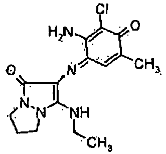 | 2-[(2-amino-3-chloro-5-methyl-4-oxocyclohexa-2,5-dien-1-ylidene)amino]-3-(ethylamino)-6,7-dihydro-1H,5H-pyrazolo[1,2-a]pyrazol-1-one |
| 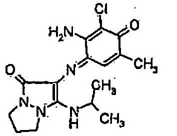 | 2-[(2-amino-3-chloro-5-methyl-4-oxocyclohexa-2,5-dien-1-ylidene)amino]-3-(isopropylamino)-6,7-dihydro-1H,5H-pyrazolo[1, 2-a]pyrazol-1-one |
| 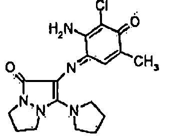 | 2-[(2-amino-3-chloro-5-methyl-4-oxocyclohexa-2,5-dien-1-ylidene)amino]-3-pyrrolidin-1-yl-6,7dihydro-1H,5H-pyrazolo[1,2-a]pyrazol-1-one |
| 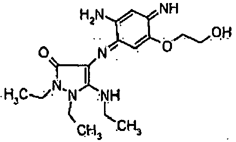 | 4-([(1E)-2-amino-5-(2-hydroxyethoxy)-4-iminocyclohexa-2,5-dien-1-ylidene]amino}-1,2-diethyl-5-(ethylamino)-1,2-dihydro-3H-pyrazol-3-one |
| 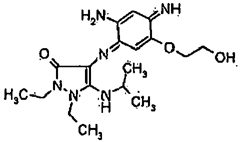 | 4-{(2-amino-5-(2-hydroxyethoxy)-4-iminocyclohexa-2,5-dien-1-ylidenelamino}-1,2-diethyl-5-(isopropylamino)-1,2-dihydro-3H-pyrazol-3-one |
| 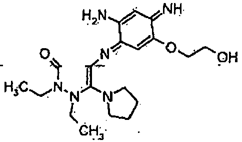 | 4-{[2-amino-5-(2-hydroxyethoxy)-4-iminocyclohexa-2,5-dien-1-ylidene]amino}-1,2-diethyl-5-pyrrolidin-1-yl-1,2-dihydro-3H-pyrazol-3-one |
| 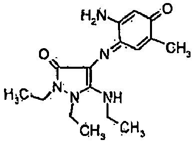 | 4-{[(1E)-2-amino-5-methyl-4-oxocyclohexa-2,5-dien-1-ylidene]amino}-1,2-diethyl-5-(elhylamino)-1,2-dihydro-3H-pyrazol-3-one |
| 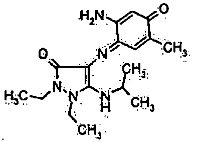 | 4-{[(1E)-2-amino-5-methyl-4-oxocyclohexa-2,5-dien-1-ylidene]amino}-1,2-diethyl-5-(isopropylamino)-1,2-dihydro-3H-pyrazol-3-one |
| 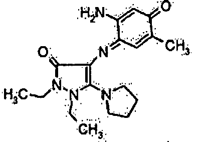 | 4-[(2-amino-5-methyl-4-oxocyclohexa-2,5-dien-1-ylidene)amino]-1,2-diethyl-5-pyrrolidin-1-yl-1,2-dihydro-3H-pyrazol-3-one |
| 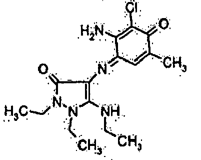 | 4-{[(1E)-2-amino-3-chloro-5-methyl-4-oxocyclohexa-2,5-dien-1-ylidene]amino}-1,2-diethyl-5-(ethylamino)-1,2-dihydro-3H-pyrazol-3-one |
| 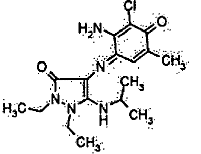 | 4-{[(1E)-2-amino-3-chloro-5-methyl-4-oxocyclohexa-2,5-dien-1-ylidene]amino}-1,2-diethyl-5-(isopropylamino)-1,2-dihydro-3H-pyrazol-3-one |
| 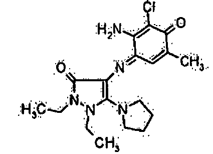 | 4-[(2-amino-3-chloro-5-methyl-4-oxocyclohexa-2,5-dien-1-ylidene)amino]-1,2-diethyl-5-pyrrolidin-1-yl-1,2-dihydro-3H-pyrazol-3-one |

## Revendications

1. Composition tinctoriale comprenant, dans un milieu approprié pour la teinture, au moins un composé choisi parmi les composés de type leuco de formule (I), les colorants de type azométhinique à motif pyrazolinone de formule (II) correspondant aux composés de formule (I), leurs formes mésomères, ainsi que leurs sels d'addition avec un acide et leurs solvates : 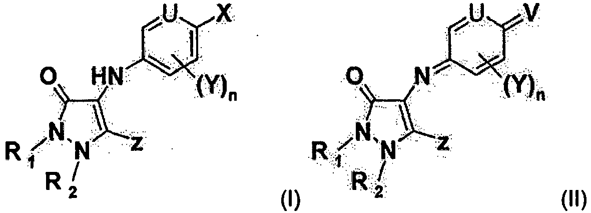 dans laquelle :
• **n** est un entier compris entre 0 et 3 ;
• **U** représente CR ou N ;
• **R** représente
- un atome d'hydrogène,
- un radical alkyle en C₁-C₄ éventuellement substitué par un radical hydroxyle ;
- un radical alcoxy en C₁-C₄ éventuellement substitué par un radical hydroxyle ;
- un radical (di)alkyl(C₁-C₄)amino dont la partie alkyle est éventuellement substituée par un radical hydroxyle ;
• **X** représente :
- un radical hydroxyle;
- un groupement NR'₁R"₁ avec R'₁ et R"₁ choisis indépendamment parmi un atome d'hydrogène ; un radical alkyle en C₁-C₆ éventuellement substitué par un ou plusieurs radicaux choisis parmi un radical hydroxy, (C₁-C₂)alcoxy, amino, (di)alkyl(C₁-C₂)amino ; un radical phényle éventuellement substitué par un ou plusieurs radicaux choisis parmi un radical hydroxy, amino, (C₁-C₂)alcoxy ;
lorsque R'₁ et R"₁ sont différents de l'hydrogène, R'₁ et R"₁ peuvent former ensemble avec l'atome d'azote auquel ils sont rattachés un hétérocycle saturé ou insaturé, comportant 5 à 7 chaînons, dont les atomes de carbone peuvent être remplacés par un atome d'oxygène ou d'azote, cet hétérocycle étant éventuellement substitué par un ou plusieurs radicaux choisis parmi un atome d'halogène, un radical amino, (di)alkyl(C₁-C₄)amino, hydroxy, carboxy, carboxamido, (C₁-C₂)alcoxy, alkyle en C₁-C₄ éventuellement substitués par un ou plusieurs radicaux choisis parmi un radical hydroxy, amino, (di)alkylamino, alcoxy, carboxy, sulfonyle ;
lorsque **X** représente un groupement NHR'₁ et que U représente un groupement CR dans lequel R désigne un radical alcoxy, alors X et U peuvent former un cycle à 6 chainons de type morpholine, éventuellement substitué par un ou plusieurs groupements alkyles C₁-C₄,
• **V** représente
∘ un atome d'oxygène
∘ un groupement NR'₁ avec R'₁ choisi parmi un atome d'hydrogène ; un radical alkyle en C₁-C₆ éventuellement substitué par un ou plusieurs radicaux choisis parmi un radical hydroxy, (C₁-C₂)alcoxy, amino, (di)alkyl(C₁-C₂)amino ; un radical phényle éventuellement substitué par un ou plusieurs radicaux choisis parmi un radical hydroxy, amino, (C₁-C₂)alcoxy
∘ un groupement N⁺R'₁R"₁ avec R'₁ et R"₁ choisis indépendamment parmi un atome d'hydrogène ; un radical alkyle en C₁-C₆ éventuellement substitué par un ou plusieurs radicaux choisis parmi un radical hydroxy, (C₁-C₂)alcoxy, amino, (di)alkyl(C₁-C₂)amino ; un radical phényle éventuellement substitué par un ou plusieurs radicaux choisis parmi un radical hydroxy, amino, (C₁-C₂)alcoxy,
lorsque V représente un groupement N⁺R'₁R"₁, l'électroneutralité de la structure (II) est assurée par un anion An-,
lorsque R'₁ et R"₁ sont différents de l'hydrogène, R'₁ et R"₁ peuvent former ensemble avec l'atome d'azote auquel ils sont rattachés un hétérocycle saturé ou insaturé, comportant 5 à 7 chaînons, dont les atomes de carbone peuvent être remplacés par un atome d'oxygène ou d'azote, cet hétérocycle étant éventuellement substitué par un ou plusieurs radicaux choisis parmi un atome d'halogène, un radical amino, (di)alkyl(C₁-C₄)amino, hydroxy, carboxy, (di)alkylcarboxamido, (C₁-C₂)alcoxy, alkyle en C₁-C₄ éventuellement substitués par un ou plusieurs radicaux choisis parmi un radical hydroxy, amino, (di)alkylamino, alcoxy, carboxy, sulfonyle ;
lorsque V représente un groupement NR'₁ et que U représente un groupement CR dans lequel R désigne un radical alcoxy, alors V et U peuvent former un cycle à 6 chainons de type morpholine, éventuellement substitué par un ou plusieurs groupements alkyles C₁-C₄,
• **Y**, identiques ou différents, représentent :
- un radical hydroxy ;
- un radical alkyle en C₁-C₄ ;
- un radical hydroxyalkyle C₁-C₄
- un atome d'halogène tel qu'un atome de chlore, d'iode, de fluor ou de brome ;
- un atome d'oxygène substitué par un radical choisi parmi un radical alkyle en C₁-C₄, un radical aryle et un radical hétéroaryle, ceux-ci pouvant être substitués par un ou plusieurs radicaux hydroxy ;
- un groupement NR'₂R'₃ ;
R'₂ et R'₃, identiques ou différents, peuvent être choisis parmi
- un atome d'hydrogène ;
- un radical alkylcarbonyle en C₁-C₄ éventuellement substitué par un groupement ammonium quaternaire tel que par exemple un trialkylammonium ou par un hétérocycle azoté cationique ou non comme par exemple un groupement imidazole, un groupement thiazole, un groupement pyridine, un groupement pipéridine, un groupement pyrrolidine, un groupement pyrimidine, un groupement pyrazine, un groupement imidazolium, un groupement pyridinium, un groupement thiazolium, un groupement pyrrolidinium, un groupement piperidinium, un groupement pyrimidinium, ces hétérocycles azotés étant eux-mêmes éventuellement substitués par un ou plusieurs radicaux alkyle en C₁-C₄ ;
- un radical aminocarbonyle ;
- un radical alkyle en C₁-C₆ éventuellement substitué par un ou plusieurs radicaux choisis parmi un radical hydroxy, (C₁-C₂)alcoxy, amino, (di)alkyl(C₁-C₂)amino ;
- un radical phényle éventuellement substitué, par un ou plusieurs radicaux choisis parmi un radical hydroxy, amino, (C₁-C₂)alcoxy ;
R'₂ et R'₃ peuvent former ensemble avec l'atome d'azote auquel ils sont rattachés un hétérocycle saturé ou insaturé, comportant 5 à 7 chaînons, dont les atomes de carbone peuvent être remplacés par un atome d'oxygène ou d'azote, cet hétérocycle étant éventuellement substitué par un ou plusieurs radicaux choisis parmi un atome d'halogène, un radical amino, (di)alkyl(C₁-C₄)amino, hydroxy, carboxy, (di)alkylcarboxamido, (C₁-C₂)alcoxy, alkyle en C₁-C₄ éventuellement substitués par un ou plusieurs radicaux choisis parmi un radical hydroxy, amino, (di)alkylamino, alcoxy, carboxy, sulfonyle ;
deux radicaux Y portés par deux atomes de carbone adjacents peuvent former ensemble avec les atomes de carbone auxquels ils sont rattachés un groupement cyclique ou hétérocyclique, saturé ou insaturé, aromatique ou non aromatique, comportant de 5 à 6 chaînons, par exemple un cycle benzène, pyrrole, pyrrolidine, pyrazole, furanne , pyrrolidine, morpholine ou imidazole, éventuellement substitué par un ou plusieurs radicaux alkyle en C₁-C₄ ;
• Z représente :
- un radical NR₃R₄ ;
- un radical OR₅ ;
• R₁, R₂, R₃, R₄ et R₅, identiques ou différents, représentent :
- un radical alkyle en C₁-C₆ éventuellement substitué par un ou plusieurs radicaux choisis parmi un radical OR₆, un radical NR₇R₈, un radical carboxy, un radical carboxylate d'alkyle en C₁-C₄, un radical sulfonique, un radical (di)alkylcarboxamido CONR₇R₈, un radical sulfonamido SO₂NR₇R₈, un radical hétéroaryle à 5 ou 6 chaînons ou phényle éventuellement substitué par un ou plusieurs radicaux choisis parmi un radical (C₁-C₄)alkyle, hydroxy, alcoxy en C₁-C₂, amino, (di)alkyl(C₁-C₂)amino ou hydroxyalkyle en C₁-C₄ ;
- un radical phényle éventuellement substitué par un ou plusieurs radicaux choisis parmi un radical (C₁-C₄)alkyle, hydroxy(C₁-C₄)alkyle, hydroxy, alcoxy en C₁-C₂, amino, (di)alkyl(C₁-C₂)amino ;
- un radical hétéroaryle à 5 ou 6 chaînons éventuellement substitué par un ou plusieurs radicaux choisis parmi un radical (C₁-C₄)alkyle, un radical (C₁-C₂)alcoxy ;
• R₃, R₄ et R₅ peuvent représenter également un atome d'hydrogène ;
• R₆, R₇ et R₈, identiques ou différents, représentent un atome d'hydrogène ; un radical alkyle linéaire ou ramifié en C₁-C₄ éventuellement substitué par un ou plusieurs radicaux choisis parmi un radical hydroxy, un radical alcoxy en C₁-C₂, un radical (di)alkylcarboxamido CONR₉R₁₀, un radical sulfonyle SO₂R₉, un radical phényle éventuellement substitué par un ou plusieurs radicaux choisis parmi un radical (C₁-C₄)alkyle, hydroxy, alcoxy en C₁-C₂, amino, (di)alkyl(C₁-C₂)amino ; un radical phényle éventuellement substitué par un ou plusieurs radicaux choisis parmi un radical (C₁-C₄)alkyle, un radical hydroxy, un radical alcoxy en C₁-C₂, un radical amino, un radical (di)alkyl(C₁-C₂)amino ;
• R₇ et R₈, identiques ou différents, peuvent représenter également un radical (di)alkylcarboxamido CONR₉R₁₀ ; un radical sulfonyle SO₂R₉ ;
• R₉ et R₁₀, identiques ou différents, représentent un atome d'hydrogène, un radical alkyle en C₁-C₄ linéaire ou ramifié éventuellement substitués par un ou plusieurs radicaux choisis parmi un radical hydroxy, un radical alcoxy en C₁-C₂ ;
• R₁ et R₂ d'une part, et R₃ et R₄ d'autre part peuvent former ensemble avec les atomes d'azote auxquels ils sont rattachés un hétérocycle saturé ou insaturé, comportant 5 à 7 chaînons, dont les atomes de carbone peuvent être remplacés par un atome d'oxygène ou d'azote, ledit cycle étant éventuellement substitué par un ou plusieurs radicaux choisis parmi un atome d'halogène, un radical amino, (di)alkyl(C₁-C₄)amino, (di)hydroxyalkyl(C₁-C₄)amino, hydroxy, carboxy, (di)alkylcarboxamido, (C₁-C₂)alcoxy, alkyle en C₁-C₄ éventuellement substitué par un ou plusieurs radicaux choisis parmi un radical hydroxy, amino, (di)alkyl(C₁-C₄)amino, alcoxy en C₁-C₂, carboxy, sulfonyle,
An- est un anion ou un mélange d'anions permettant d'assurer l'électroneutralité des structures.

2. Composition selon la revendication 1 dans laquelle dans la formule (I), les radicaux R₁ et R₂, identiques ou différents, sont choisis parmi :
- un radical alkyle en C₁-C₄ éventuellement substitué par un ou plusieurs radicaux choisis parmi un radical hydroxy, un radical (C₁-C₂)alcoxy, un radical amino, un radical (di)alkyl(C₁-C₂)amino ;
- un radical phényle éventuellement substitué par un ou plusieurs radicaux choisis parmi un radical alkyle en C₁-C₄, un radical hydroxyalkyle en C₁-C₄.

3. Composition selon la revendication 1 dans laquelle les radicaux R₁ et R₂ forment ensemble avec les atomes d'azote auxquels ils sont rattachés un cycle à 5 ou 6 chaînons, saturé ou insaturé, éventuellement substitué.

4. Composition selon l'une quelconque des revendications précédentes dans laquelle les composés (I) ou (II) sont tels que Z représente un radical NR₃R₄.

5. Composition selon la revendication 1 dans laquelle Z représente un radical NR₃R₄ et les radicaux R₃ et R₄ forment ensemble avec l'atome d'azote auquel ils sont rattachés un cycle de 5 à 7 chaînons choisi parmi les hétérocycles pyrrolidine, pipéridine, homopipéridine, pipérazine, homopipérazine, morpholine ; lesdits cycles pouvant être substitués par un ou plusieurs radicaux choisis parmi un radical hydroxy, amino, (di)alkyl(C₁-C₂)amino, (di)hydroxyalkyl(C₁-C₂)amino, (di)alkyl(C₁-C₂)carboxamido, carboxy, alkyle en C₁-C₄ éventuellement substitué par un ou plusieurs radicaux choisis parmi un radical hydroxy, amino, (di)alkylamino en C₁-C₂.

6. Composition selon l'une quelconque des revendications précédentes dans laquelle U représente CR ou N, et R représente un atome d'hydrogène, un radical méthyle, un radical méthoxy, un radical 2-hydroxyéthoxy, un radical méthylamino, un radical diméthylamino ou hydroxyéthylamino ou un radical dihydroxyéthylamino ou un radical méthyl(hydroxyéthyl)amino.

7. Composition selon l'une quelconque des revendications précédentes dans laquelle X représente un radical hydroxyle ; un groupement NR'₁R"₁ avec R'₁ et R"₁ choisis indépendamment parmi un atome d'hydrogène ; un radical alkyle en C₁-C₆ éventuellement substitué par un ou plusieurs radicaux hydroxyle.

8. Composition selon l'une quelconque des revendications précédentes dans laquelle X représente un groupement NR'₁R"₁ avec R'1 et R"1 formant un hétérocycle choisi parmi les hétérocycles pyrrolidine, pipéridine, homopipéridine, pipérazine, homopipérazine, morpholine ; les dits cycles pouvant être substitués par un ou plusieurs radicaux choisis parmi un atome d'halogène, un radical amino, (di)alkyl(C₁-C₄)amino, hydroxy, carboxy, (di)alkylcarboxamido, (C₁-C₂)alcoxy, alkyle en C₁-C₄ éventuellement substitués par un ou plusieurs radicaux choisis parmi un radical hydroxy, amino, (di)alkylamino, alcoxy, carboxy, sulfonyle.

9. Composition selon l'une quelconque des revendications précédentes dans laquelle les composés de formule (I) et (II) sont tels que V représente un atome d'oxygène, un groupement NR'₁ dans lequel R'₁ représente un atome d'hydrogène ou un radical alkyle en C₁-C₆ éventuellement substitué par un ou plusieurs radicaux hydroxyle, un groupement N+R'1R"1 dans lequel R'1 et R"1 sont choisis indépendamment parmi un atome d'hydrogène, un radical alkyle en C1-C6 éventuellement substitué par un ou plusieurs radicaux hydroxy, ou dans lequel R'₁ et R"1 peuvent former ensemble avec l'atome d'azote auxquels ils sont rattachés un hétérocycle saturé ou insaturé, comportant de 5 à 7 chainons.

10. Composition selon l'une quelconque des revendications précédentes dans laquelle Y, identiques ou différents, représentent un radical hydroxy ; un radical alkyle en C₁-C₄ ; un atome d'halogène ; un atome d'oxygène substitué par un radical alkyle en C₁-C₄ pouvant être substitué par un ou plusieurs radicaux hydroxy ; un groupement NR'₂R'₃ ; R'₂ et R'₃, identiques ou différents, peuvent être choisis parmi un atome d'hydrogène ; un radical alkylcarbonyle en C₁-C₄ éventuellement substitué par par un groupement ammonium quaternaire ou R'₂ et R'3 peuvent former ensemble avec l'atome d'azote auquel ils sont rattachés un hétérocycle saturé comportant de 5 à 7 chaînons.

11. Composition selon l'une quelconque des revendications précédentes **caractérisée en ce qu'**elle comprend également un agent oxydant.

12. Composés choisis parmi les composés de type leuco de formule (I), les colorants de type azométhinique à motif pyrazolinone de formule (II) correspondant aux composés de formule (I) tels que définis à l'une quelconque des revendications 1 à 10, leurs formes mésomères, ainsi que leurs sels d'addition avec un acide et leurs solvates ;

13. Procédé de teinture des fibres kératiniques **caractérisé par le fait que** l'on applique sur ces fibres au moins une composition tinctoriale comprenant au moins un composé choisi parmi les composés de type leuco de formule (I), les colorants de type azométhinique à motif pyrazolinone de formule (II) correspondant aux composés de formule (I) tels que définis à l'une quelconque des revendications 1 à 10, leurs formes mésomères, ainsi que leurs sels d'addition avec un acide et leurs solvates ;.

14. Dispositif à plusieurs compartiments, ou "kit" de teinture à plusieurs compartiments, dont un premier compartiment contient au moins un composé de formule (I), tel que défini à l'une quelconque des revendications 1 à 10, et un deuxième compartiment contient un agent oxydant, et éventuellement un composé de formule (II), tel que défini à l'une quelconque des revendications 1 à 10, un agent alcalin.

## Patentansprüche

1. Färbezusammensetzung, die in einem für die Färbung geeigneten Medium mindestens eine Verbindung umfasst, die aus den Verbindungen vom Leuko-Typ der Formel (I), den Farbstoffen vom Azomethin-Typ mit einer Pyrazolinon-Einheit der Formel (II), die den Verbindungen der Formel (I) entsprechen, deren mesomeren Formen sowie deren Säureadditionssalzen und deren Solvaten ausgewählt ist: 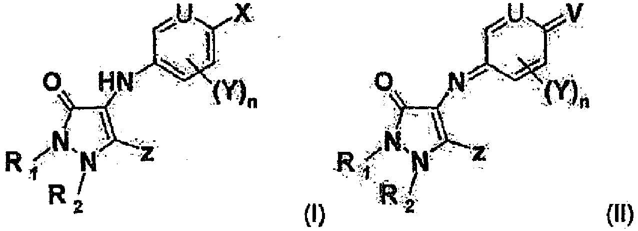 worin:
• n für eine ganze Zahl zwischen 0 und 3 steht;
• U für CR oder N steht;
• R für:
- ein Wasserstoffatom;
- einen C₁-C₄-Alkylrest,der gegebenenfalls durch einen Hydroxylrest substituiert ist;
- einen C₁-C₄-Alkoxyrest, der gegebenenfalls durch einen Hydroxylrest substituiert ist;
- einen (Di)alkyl(C₁-C₄₎aminorest, dessen Alkylteil gegebenenfalls durch einen Hydroxylrest substituiert ist;
steht;
• X für;
- einen Hydroxylrest;
- eine NR'₁R' '₁-Gruppe, wobei R'₁ und R' '₁ unabhängig voneinander aus einem Wasserstoffatom; einem C₁-C₆-Alkylrest, der gegebenenfalls durch einen oder mehrere aus einem Hydroxy- , (C₁-C₂)Alkoxy-, Amino- und (Di) alkyl (C₁-C₂) aminorest ausgewählte Reste substituiert ist; und einem Phenylrest, der gegebenenfalls durch einen oder mehrere aus einem Hydroxy-, Amino- und (C₁-C₂)Alkoxyrest ausgewählte Reste substituiert ist; ausgewählt sind;
steht;
dann, wenn R'₁ und R' '1 von Wasserstoff verschieden sind, R'₁ und R''₁ zusammen mit dem Stickstoffatom, an das sie gebunden sind, einen gesättigten oder ungesättigten 5- bis 7-gliedrigen Heterocyclus, dessen Kohlenstoffatome durch ein Sauerstoff- oder Stickstoffatom ersetzt sein können, bilden können, wobei dieser Heterocyclus gegebenenfalls durch einen oder mehrere aus einem Halogenatom und einem Amino-, (Di)alkyl(C₁-C₄)amino-, Hydroxy-, Carboxy-, Carboxamido-, (C₁-C₂)Alkoxy- und C₁-C₄-Alkylrest ausgewählte Reste, die gegebenenfalls durch einen oder mehrere Reste, die aus einem Hydroxy-, Amino-, (Di)alkylamino-, Alkoxy-, Carboxy- und Sulfonylrest ausgewählt sind, substituiert sein können, substituiert ist;
dann, wenn X für eine NHR'₁-Gruppe steht und U für eine CR-Gruppe, in der R für einen Alkoxyrest steht, X und U einen 6-gliedrigen Ring vom Morpholin-Typ, der gegebenenfalls durch eine oder mehrere C₁-C₄-Alkylgruppen substituiert ist, bilden können;
• V für:
∘ ein Sauerstoffatom;
∘ eine NR'₁-Gruppe, wobei R'₁ aus einem Wasserstoffatom; einem C₁-C₆-Alkylrest, der gegebenenfalls durch einen oder mehrere aus einem Hydroxy-, (C₁-C₂) Alkoxy-, Amino- und (Di)alkyl(C₁-C₂) aminorest ausgewählte Reste substituiert ist; und einem Phenylrest, der gegebenenfalls durch einen oder mehrere aus einem Hydroxy-, Amino- und (C₁-C₂)Alkoxyrest ausgewählte Reste substituiert ist; ausgewählt ist;
∘ eine N⁺R' ₁R' '₁-Gruppe, wobei R' ₁ und R"₁ unabhängig voneinander aus einem Wasserstoffatom; einem C₁-C₆-Alkylrest, der gegebenenfalls durch einen oder mehrere aus einem Hydroxy-, (C₁-C₂)Alkoxy-, Amino- und (Di)-alkyl (C₁-C₂) aminorest ausgewählte Reste substituiert ist; und einem Phenylrest, der gegebenenfalls durch einen oder mehrere aus einem Hydroxy-, Amino- und (C₁-C₂)Alkoxyrest ausgewählte Reste substituiert ist; ausgewählt sind;
steht;
dann, wenn V für eine N⁺R'₁R''₁-Gruppe steht, die Elektroneutralität der Struktur (II) durch ein Anion An- gewährleistet ist, dann, wenn R'₁ und R"₁ von Wasserstoff verschieden sind, R'₁ und R"₁ zusammen mit dem Stickstoffatom, an das sie gebunden sind, einen gesättigten oder ungesättigten 5- bis 7-gliedrigen Heterocyclus, dessen Kohlenstoffatome durch ein Sauerstoff- oder Stickstoffatom ersetzt sein können, bilden können, wobei dieser Heterocyclus gegebenenfalls durch einen oder mehrere aus einem Halogenatom und einem Amino-, (Di)alkyl (C₁-C₄)amino-, Hydroxy-, Carboxy-, Carboxamido-, (C₁-C₂)Alkoxy- und C₁-C₄-Alkylrest ausgewählte Reste, die gegebenenfalls durch einen oder mehrere Reste, die aus einem Hydroxy-, Amino-, (Di)alkylamino-, Alkoxy-, Carboxy- und Sulfonylrest ausgewählt sind, substituiert sein können, substituiert ist;
dann, wenn V für eine NR'₁-Gruppe steht und U für eine CR-Gruppe, in der R für einen Alkoxyrest steht, V und U einen 6-gliedrigen Ring vom Morpholin-Typ, der gegebenenfalls durch eine oder mehrere C₁-C₄-Alkylgruppen substituiert ist, bilden können;
• die Y-Reste gleich oder verschieden sind und für:
einen Hydroxyrest;
- einen C₁-C₄-Alkylrest;
- einen C₁-C₄-Hydroxyalkylrest;
- ein Halogenatom wie ein Chlor-, Iod-, Fluor- oder Bromatom;
- ein Sauerstoffatom, das durch einen Rest, der aus einem C₁-C₄-Alkylrest, einem Arylrest und einem Heteroarylrest ausgewählt ist, substituiert ist, wobei diese Reste durch einen oder mehrere Hydroxyreste substituiert sein können;
- eine NR' ₂R' ₃-Gruppe
stehen;
R'₂ und R'₃ gleich oder verschieden sind und aus:
- einem Wasserstoffatom;
- einem C₁-C₄-Alkylcarbonylrest, der gegebenenfalls durch eine quartäre Ammoniumgruppe wie beispielsweise ein Trialkylammonium oder durch einen kationischen oder nichtkationischen stickstoffhaltigen Heterocyclus wie beispielsweise eine Imidazolgruppe, eine Thiazolgruppe, eine Pyridingruppe, eine Piperidingruppe, eine Pyrrolidingruppe, eine Pyrimidingruppe, eine Pyrazingruppe, eine Imidazoliumgruppe, eine Pyridiniumgruppe, eine Thiazoliumgruppe, eine Pyrrolidiniumgruppe, eine Piperidiniumgruppe oder eine Pyrimidiniumgruppe substituiert ist, wobei diese stickstoffhaltigen Heterocyclen selbst gegebenenfalls durch einen oder mehrere C₁-C₄-Alkylreste substituiert sind;
- einem Aminocarbonylrest;
- einem C₁-C₆-Alkylrest, der gegebenenfalls durch einen oder mehrere aus einem Hydroxy-, (C₁-C₂)Alkoxy-, Amino- und (Di)alkyl(C₁-C₂)aminorest ausgewählte Reste substituiert ist;
- einem Phenylrest, der gegebenenfalls durch einen oder mehrere aus einem Hydroxy-, Amino- und (C₁-C₂) Alkoxyrest ausgewählte Reste substituiert ist;
ausgewählt sein können;
R'₂ und R'₃ zusammen mit dem Stickstoffatom, an das sie gebunden sind, einen gesättigten oder ungesättigten 5- bis 7-gliedrigen Heterocyclus, dessen Kohlenstoffatome durch ein Sauerstoff- oder Stickstoffatom ersetzt sein können, bilden können, wobei dieser Heterocyclus gegebenenfalls durch einen oder mehrere aus einem Halogenatom und einem Amino-, (Di)alkyl(C₁-C₄)amino-, Hydroxy-, Carboxy-, (Di)alkylcarboxamido-, (C₁-C₂)Alkoxy- und C₁-C₄-Alkylrest ausgewählte Reste, die gegebenenfalls durch einen oder mehrere Reste, die aus einem Hydroxy-, Amino-, (Di)alkylamino-, Alkoxy-, Carboxy- und Sulfonylrest ausgewählt sind, substituiert sein können, substituiert ist;
zwei Y-Reste an zwei benachbarten Kohlenstoffatomen zusammen mit den Kohlenstoffatomen, an die sie gebunden sind, eine cyclische oder heterocyclische, gesättigte oder ungesättigte, aromatische oder nichtaromatische 5- bis 6-gliedrige Gruppe, beispielsweise einen Benzol-, Pyrrol-, Pyrrolidin-, Pyrazol-, Furan-, Pyrrolidin-, Morpholin- oder Imidazolring, der gegebenenfalls durch einen oder mehrere C₁-C₄-Alkylreste substituiert ist, bilden können.
• Z für:
- einen NR₃R₄-Rest oder
- einen OR₅-Rest
steht;
• R_{1,} R₂, R₃, R₄ und R₅ gleich oder verschieden sind und für:
- einen C₁-C₆-Alkylrest, der gegebenenfalls durch einen oder mehrere aus einem OR₆-Rest, einem NR₇R₈-Rest, einem Carboxyrest, einem C₁-C₄-Alkyl-carboxylatrest, einem Sulfonsäurerest, einem (Di)-alkylcarboxamidorest CONR₇R₈, einem Sulfonamidorest SO₂NR₇R₈ und einem 5- oder 6-gliedrigen Heteroaryl- oder Phenylrest, der gegebenenfalls durch einen oder mehrere aus einem (C₁-C₄)Alkyl-, Hydroxy-, C₁-C₂-Alkoxy-, Amino-, (Di)alkyl (C₁-C₂)amino- und C₁-C₄-Hydroxyalkylrest ausgewählte Reste substituiert ist; ausgewählt sind;
- einen Phenylrest, der gegebenenfalls durch einen oder mehrere aus einem (C₁-C₄)Alkyl-, Hydroxy(C₁-C₄)alkyl-, Hydroxy-, Alkoxy-, Amino- und (Di)(C₁-C₂)alkylaminorest ausgewählte Reste substituiert ist;
- einen 5- bis 6-gliedrigen Heteroarylrest, der gegebenenfalls durch einen oder mehrere aus einem (C₁-C₄) Alkylrest und einem (C₁-C₂) -Alkoxyrest ausgewählte Reste substituiert ist;
stehen;
• R₃, R₄ und R₅ auch für ein Wasserstoffatom stehen können;
• R₆, R₇ und R₈ gleich oder verschieden sind und für ein Wasserstoffatom; einen linearen oder verzweigten C₁-C₄-Alkylrest, der gegebenenfalls durch einen oder mehrere aus einem Hydroxyrest, einem C₁-C₂-Alkoxyrest, einem (Di)alkylcarboxamidorest CONR₉R₁₀, einem Sulfonylrest SO₂R₉ und einem Phenylrest, der gegebenenfalls durch einen oder mehrere aus einem (C₁-C₄)Alkyl-, Hydroxy-, C₁-C₂-Alkoxy-, Amino- und (Di)(C₁-C₂)alkylaminorest ausgewählte Reste substituiert ist; oder einen Phenylrest, der gegebenenfalls durch einen oder mehrere aus einem (C₁-C₄) Alkylrest, einem Hydroxyrest, einem C₁-C₂-Alkoxyrest, einem Aminorest und einem (Di)alkyl(C₁-C₂)aminorest ausgewählte Reste substituiert ist; stehen;
• R₇ und R₈ gleich oder verschieden sind und auch für einen (Di)alkylcarboxamidorest CONR₉R₁₀ oder einen Sulfonylrest SO₂R₉ stehen können;
• R₉ und R₁₀ gleich oder verschieden sind und für ein Wasserstoffatom oder einen linearen oder verzweigten C₁-C₄-Alkylrest, der gegebenenfalls durch einen oder mehrere aus einem Hydroxyrest und einem C₁-C₂-Alkoxyrest ausgewählte Reste substituiert ist, stehen;
• R₁ und R₂ einerseits und R₃ und R₄ andererseits zusammen mit den Stickstoffatomen, an die sie gebunden sind, einen gesättigten oder ungesättigten 5- bis 7-gliedrigen Heterocyclus, dessen Kohlenstoffatome durch ein Sauerstoff- oder Stickstoffatom ersetzt sein können, bilden können, wobei dieser Cyclus gegebenenfalls durch einen oder mehrere aus einem Halogenatom und einem Amino-, (Di)alkyl(C₁-C₄) amino-, (Di)hydroxyalkyl-(C₁-C₄) amino-, Hydroxy-, Carboxy-, (Di)alkylcarboxamido-, (C₁-C₂)Alkoxy- und C₁-C₄-Alkylrest, der gegebenenfalls durch einen oder mehrere aus einem Hydroxy- , Amino-, (Di)alkyl(C₁-C₄) amino-, C₁-C₂-Alkoxy-, Carboxy- und Sulfonylrest ausgewählte Reste substituiert ist, ausgewählte Reste substituiert ist,
An- für ein Anion oder eine Mischung von Anionen, das bzw. die die Gewährleistung der Elektroneutralität der Derivate der Formel (I) und (II) erlaubt, steht.

2. Zusammensetzung nach Anspruch 1, wobei in der Formel (I) die Reste R₁ und R₂ gleich oder verschieden sind und aus:
- einem C₁-C₄-Alkylrest, der gegebenenfalls durch einen oder mehrere aus einem Hydroxyrest, einem (C₁-C₂)Alkoxyrest, einem Aminorest und einem (Di)alkyl(C₁-C₂)aminorest ausgewählte Reste substituiert ist; und
- einem Phenylrest, der gegebenenfalls durch einen oder mehrere aus einem C₁-C₄-Alkylrest und einem C₁-C₄-Hydroxyalkylrest ausgewählte Reste substituiert ist;
ausgewählt sind.

3. Zusammensetzung nach Anspruch 1, wobei die Reste R₁ und R₂ zusammen mit den Stickstoffatomen, an die sie gebunden sind, einen gesättigten oder ungesättigten 5- oder 6-gliedrigen Ring, der gegebenenfalls substituiert ist, bilden.

4. Zusammensetzung nach einem der vorhergehenden Ansprüche, wobei die Verbindungen der Formel (I) oder (II) so beschaffen sind, dass Z für einen NR₃R₄-Rest steht.

5. Zusammensetzung nach Anspruch 1, wobei Z für einen NR₃R₄-Rest steht und die Reste R₃ und R₄ zusammen mit dem Stickstoffatom, an das sie gebunden sind, einen 5- bis 7-gliedrigen Heterocyclus, bilden, der aus Pyrrolidin-, Piperidin-, Homopiperidin-, Piperazin-, Homopiperazin- und Morpholin-Heterocyclen ausgewählt ist; wobei diese Cyclen gegebenenfalls durch einen oder mehrere aus einem Hydroxy-, Amino-, (Di)alkyl (C₁-C₂) amino-, (Di)-hydroxyalkyl (C₁-C₂) amino-, (Di)alkyl(C₁-C₂) carboxamido-, Carboxy- und C₁-C₄-Alkylrest, der gegebenenfalls durch einen oder mehrere aus einem Hydroxy-, Amino- und C₁-C₂-(Di)alkylaminorest ausgewählte Reste substituiert ist, ausgewählte Reste substituiert sind.

6. Zusammensetzung nach einem der vorhergehenden Ansprüche, wobei U für CR oder N steht und R für ein Wasserstoffatom, einen Methylrest, einen Methoxyrest, einen 2-Hydroxyethoxyrest, einen Methylaminorest, einen Dimethylamino- oder Hydroxyethylaminorest oder einen Dihydroxyethylaminorest oder einen Methyl(hydroxyethyl)aminorest steht.

7. Zusammensetzung nach einem der vorhergehenden Ansprüche, wobei X für einen Hydroxylrest oder eine NR'₁R''₁-Gruppe steht, wobei R'₁ und R''₁ unabhängig voneinander aus einem Wasserstoffatom und einem C₁-C₆-Alkylrest, der gegebenenfalls durch einen oder mehrere Hydroxylreste substituiert ist; ausgewählt sind.

8. Zusammensetzung nach einem der vorhergehenden Ansprüche, wobei X für einen Hydroxylrest oder eine NR'₁R''₁-Gruppe steht, wobei R'₁ und R''₁ einen Heterocyclus bilden, der aus Pyrrolidin-, Piperidin-, Homopiperidin-, Piperazin-, Homopiperazin- und Morpholin-Heterocyclen ausgewählt ist; wobei diese Cyclen gegebenenfalls durch einen oder mehrere aus einem Halogenatom und einem Amino-, (Di)alkyl(C₁-C₄)amino-, Hydroxy-, Carboxy-, (Di)alkylcarboxamido-, (C₁-C₂)Alkoxy und C₁-C₄-Alkylrest, der gegebenenfalls durch einen oder mehrere aus einem Hydroxy-, Amino-, (Di)alkylamino-, Alkoxy-, Carboxy- und Sulfonylrest ausgewählte Reste substituiert ist, ausgewählte Reste substituiert sind.

9. Zusammensetzung nach einem der vorhergehenden Ansprüche, wobei die Verbindungen der Formel (I) und (II) so beschaffen sind, dass V für ein Sauerstoffatom; eine NR'₁-Gruppe steht, wobei R'₁ für ein Wasserstoffatom oder einen C₁-C₆-Alkylrest, der gegebenenfalls durch einen oder mehrere Hydroxylreste substituiert ist, oder eine N⁺R'₁R''₁-Gruppe, wobei R'₁ und R"₁ unabhängig voneinander aus einem Wasserstoffatom und einem C₁-C₆-Alkylrest, der gegebenenfalls durch einen oder mehrere Hydroxyreste substituiert ist, ausgewählt sind oder wobei R'₁ und R"₁ zusammen mit dem Stickstoffatom, an das sie gebunden sind, einen gesättigten oder ungesättigten 5- bis 7-gliedrigen Heterocyclus bilden können, steht.

10. Zusammensetzung nach einem der vorhergehenden Ansprüche, wobei die Y-Reste gleich oder verschieden sind und für einen Hydroxyrest; einen C₁-C₄-Alkylrest; ein Halogenatom; ein Sauerstoffatom, das durch einen C₁-C₄-Alkylrest, der durch einen oder mehrere Hydroxyreste substituiert sein kann, substituiert ist; oder eine NR'₂R'₃-Gruppe stehen und R'₂ und R'₃ gleich oder verschieden sind und aus einem Wasserstoffatom und einem C₁-C₄-Alkylcarbonylrest der gegebenenfalls durch eine quaternäre Ammoniumgruppe substituiert ist, ausgewählt sind oder R'₂ und R'₃ zusammen mit dem Stickstoffatom, an das sie gebunden sind, einen gesättigten 5- bis 7-gliedrigen Heterocyclus bilden können.

11. Zusammensetzung nach einem der vorhergehenden Ansprüche, **dadurch gekennzeichnet, dass** sie außerdem ein Oxidationsmittel umfasst.

12. Verbindungen, die aus den Verbindungen vom Leuko-Typ der Formel (I), den Farbstoffen vom Azomethin-Typ mit einer Pyrazolinon-Einheit der Formel (II), die den Verbindungen der Formel (I) entsprechen, deren mesomeren Formen sowie deren Säureadditionssalzen und deren Solvaten ausgewählt sind.

13. Verfahren zum Färben von Keratinfasern, **dadurch gekennzeichnet, dass** man auf diese Fasern mindestens eine Färbezusammensetzung, die mindestens eine Verbindung umfasst, die aus den Verbindungen vom Leuko-Typ der Formel (I), den Farbstoffen vom Azomethin-Typ mit einer Pyrazolinon-Einheit der Formel (II), die den Verbindungen der Formel (I) entsprechen, gemäß der in einem der Ansprüche 1 bis 10 angegebenen Definition, deren mesomeren Formen sowie deren Säureadditionssalzen und deren Solvaten ausgewählt ist, aufbringt.

14. Vorrichtung mit mehreren Kompartimenten oder Färbe-"Kit" mit mehreren Kompartimenten, wobei ein erstes Kompartiment mindestens eine Verbindung der Formel (I) gemäß der in einem der Ansprüche 1 bis 10 angegebenen Definition enthält und ein zweites Kompartiment ein Oxidationsmittel und gegebenenfalls eine Verbindung der Formel (II) gemäß der in einem der Ansprüche 1 bis 10 angegebenen Definition und ein alkalisches Mittel enthält.

## Claims

1. Dyeing composition comprising, in a medium appropriate for dyeing, at least one compound selected from the leuco compounds of formula (I), the azomethine dyes with a pyrazolinone unit of formula (II) corresponding to the compounds of formula (I), their mesomeric forms, and also their addition salts with an acid and their solvates: 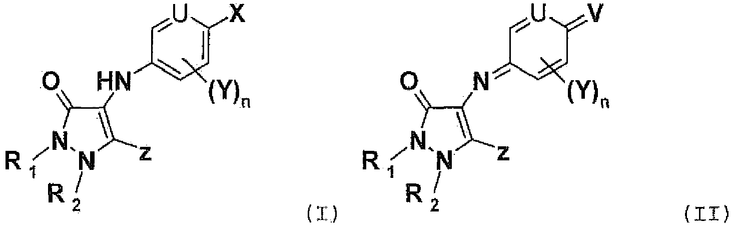 in which:
• **n** is an integer between 0 and 3;
• **U** represents CR or N;
• **R** represents
- a hydrogen atom,
- a C₁-C₄ alkyl radical optionally substituted by a hydroxyl radical,
- a C₁-C₄ alkoxy radical optionally substituted by a hydroxyl radical;
- a (di)alkyl(C₁-C₄) amino radical whose alkyl moiety is optionally substituted by a hydroxyl radical;
• **X** represents:
- a hydroxyl radical;
- a group NR'₁R"₁ with R'₁ and R"₁ selected independently from a hydrogen atom; a C₁-C₆ alkyl radical optionally substituted by one or more radicals selected from a hydroxyl, (C₁-C₂)alkoxy, amino, (di)alkyl(C₁-C₂)amino radical; a phenyl radical optionally substituted by one or more radicals selected from a hydroxyl, amino, (C₁-C₂)alkoxy radical;
when R'₁ and R"₁ are other than hydrogen, R'₁ and R"₁ may form, together with the nitrogen atom to which they are attached, a saturated or unsaturated heterocycle, containing 5 to 7 members, whose carbon atoms may be replaced by an oxygen or nitrogen atom, this heterocycle being optionally substituted by one or more radicals selected from a halogen atom, an amino, (di)alkyl(C₁-C₄)amino, hydroxyl, carboxyl, carboxamide, (C₁-C₂)alkoxy, C₁-C₄alkyl radical which are optionally substituted by one or more radicals selected from a hydroxyl, amino, (di)alkylamino, alkoxy, carboxyl, sulfonyl radical;
when **X** represents a group NHR'₁ and when U represents a group CR in which R denotes an alkoxy radical, then X and U may form a 6-membered ring of morpholine type which is optionally substituted by one or more C₁-C₄ alkyl groups,
• **V** represents
∘ an oxygen atom
∘ a group NR'₁ with R'₁ selected from a hydrogen atom; a C₁-C₆ alkyl radical optionally substituted by one or more radicals selected from a hydroxyl, (C₁-C₂)alkoxy, amino, (di)alkyl(C₁-C₂)amino radical ; a phenyl radical optionally substituted by one or more radicals selected from a hydroxyl, amino, (C₁-C₂)alkoxy radical,
∘ a group N⁺R'₁R"₁ with R'₁ and R"₁ selected independently from a hydrogen atom; a C₁-C₆ alkyl radical optionally substituted by one or more radicals selected from a hydroxyl, (C₁-C₂) alkoxy, amino, (di)alkyl(C₁-C₂) amino radical; a phenyl radical optionally substituted by one or more radicals selected from a hydroxyl, amino, (C₁-C₂)alkoxy radical,
when V represents a group N⁺R'₁R"₁, the electroneutrality of the structure (II) is ensured by an anion An-,
when R'₁ and R"₁ are other than hydrogen, R'₁ and R"₁ may form, together with the nitrogen atom to which they are attached, a saturated or unsaturated heterocycle, containing 5 to 7 members, whose carbon atoms may be replaced by an oxygen or nitrogen atom, this heterocycle being optionally substituted by one or more radicals selected from a halogen atom, an amino, (di)alkyl(C₁-C₄)amino, hydroxyl, carboxyl, (di)alkylcarboxamido, (C₁-C₂)alkoxy, C₁-C₄ alkyl radical which are optionally substituted by one or more radicals selected from a hydroxyl, amino, (di)alkylamino, alkoxy, carboxyl, sulfonyl radical;
when V represents a group NR'₁ and when U represents a group CR in which R denotes an alkoxy radical, then V and U may form a 6-membered ring of morpholine type which is optionally substituted by one or more C₁-C₄ alkyl groups,
• **Y**, which are identical or different, represent:
- a hydroxyl radical;
- a C₁-C₄alkyl radical;
- a C₁-C₄hydroxyalkyl radical;
- a halogen atom such as a chlorine, iodine, fluorine or bromine atom;
- an oxygen atom substituted by a radical selected from a C₁-C₄ alkyl radical, an aryl radical and a heteroaryl radical, it being possible for these to be substituted by one or more hydroxyl radicals;
- a group NR'₂R'₃;
R'₂ and R'₃, which are identical or different, may be selected from
- a hydrogen atom;
- a C₁-C₄ alkylcarbonyl radical optionally substituted by a quaternary ammonium group such as, for example, a trialkylammonium or by a cationic or non-cationic nitrogen-containing heterocycle such as, for example, an imidazole group, a thiazole group, a pyridine group, a piperidine group, a pyrrolidine group, a pyrimidine group, a pyrazine group, an imidazolium group, a pyridinium group, a thiazolium group, a pyrrolidinium group, a piperidinium group, a pyrimidinium group, these nitrogen-containing heterocycles being themselves optionally substituted by one or more C₁-C₄ alkyl radicals;
- an aminocarbonyl radical;
- a C₁-C₆ alkyl radical optionally substituted by one or more radicals selected from a hydroxyl, (C₁-C₂)alkoxy, amino, (di)alkyl(C₁-C₂)amino radical;
- a phenyl radical optionally substituted by one or more radicals selected from a hydroxyl, amino, (C₁-C₂)alkoxy radical;
R'₂ and R'₃ may form, together with the nitrogen atom to which they are attached, a saturated or unsaturated heterocycle, containing 5 to 7 members, whose carbon atoms may be replaced by an oxygen or nitrogen atom, this heterocycle being optionally substituted by one or more radicals selected from a halogen atom, an amino, (di)alkyl(C₁-C₄)amino, hydroxyl, carboxyl, (di)alkylcarboxamido, (C₁-C₂)alkoxy, C₁-C₄ alkyl radical which are optionally substituted by one or more radicals selected from a hydroxyl, amino, (di)alkylamino, alkoxy, carboxyl, sulfonyl radical;
two radicals Y carried by two adjacent carbon atoms may form, together with the carbon atoms to which they are attached, a saturated or unsaturated, aromatic or nonaromatic, cyclic or heterocyclic group containing 5 to 6 members, for example a benzene, pyrrole, pyrrolidine, pyrazole, furan, pyrrolidine, morpholine or imidazole ring, optionally substituted by one or more C₁-C₄alkyl radicals;
• Z represents:
- a radical NR₃R₄;
- a radical OR₅;
• R₁, R₂, R₃, R₄ and R₅, which are identical or different, represent:
- a C₁-C₆ alkyl radical optionally substituted by one or more radicals selected from a radical OR₆, a radical NR₇R₈, a carboxyl radical, a C₁-C₄ alkyl carboxylate radical, a sulfonic radical, a (di)alkylcarboxamido radical CONR₇R₈, a sulfonamido radical SO₂NR₇R₈, a 5- or 6-membered heteroaryl or phenyl radical optionally substituted by one or more radicals selected from a (C₁-C₄) alkyl, hydroxyl, C₁-C₂ alkoxy, amino, (di) alkyl (C₁-C₂) amino or C₁-C₄ hydroxyalkyl radical;
- a phenyl radical optionally substituted by one or more radicals selected from a (C₁-C₄)alkyl, hydroxy (C₁-C₄) alkyl, hydroxyl, C₁- C₂ alkoxy, amino, (di) alkyl (C₁-C₂) amino radical;
- a 5- or 6-membered heteroaryl radical optionally substituted by one or more radicals selected from a (C₁-C₄) alkyl radical, a (C₁-C₂) alkoxy radical;
• R₃, R₄ and R₅ may also represent a hydrogen atom;
• R₆, R₇ and R₈, which are identical or different, represent a hydrogen atom; a linear or branched C₁-C₄ alkyl radical optionally substituted by one or more radicals selected from a hydroxyl radical, a C₁-C₂ alkoxy radical, a (di)alkylcarboxamido radical CONR₉R₁₀, a sulfonyl radical SO₂R₉, a phenyl radical optionally substituted by one or more radicals selected from a (C₁-C₄) alkyl, hydroxyl, C₁-C₂ alkoxy, amino, (di)alkyl(C₁-C₂)amino radical; a phenyl radical optionally substituted by one or more radicals selected from a (C₁-C₄)alkyl radical, a hydroxyl radical, a C₁-C₂ alkoxy radical, an amino radical, a (di)alkyl(C₁-C₂)amino radical;
• R₇ and R₈, which are identical or different, may also represent a (di)alkylcarboxamido radical CONR₉R₁₀; a sulfonyl radical SO₂R₉;
• R₉ and R₁₀, which are identical or different, represent a hydrogen atom, a linear or branched C₁-C₄ alkyl radical optionally substituted by one or more radicals selected from a hydroxyl radical, a C₁-C₂ alkoxy radical;
• R₁ and R₂, on the one hand, and R₃ and R₄, on the other hand, may form, together with the nitrogen atoms to which they are attached, a saturated or unsaturated heterocycle, containing 5 to 7 members, whose carbon atoms may be replaced by an oxygen or nitrogen atom, said heterocycle being optionally substituted by one or more radicals selected from a halogen atom, an amino, (di)alkyl(C₁-C₄) amino, (di)hydroxyalkyl(C₁-C₄) amino, hydroxyl, carboxyl, (di)alkylcarboxamido, (C₁-C₂)alkoxy, C₁-C₄ alkyl radical optionally substituted by one or more radicals selected from a hydroxyl, amino, (di) alkyl (C₁-C₄) amino, C₁-C₂ alkoxy, carboxyl, sulfonyl radical,
An- is an anion or a mixture of anions which allows the electroneutrality of the structures to be ensured.

2. Composition according to Claim 1, in which in the formula (I) the radicals R₁ and R₂, which are identical or different, are selected from:
- a C₁-C₄ alkyl radical optionally substituted by one or more radicals selected from a hydroxyl radical, a (C₁-C₂)alkoxy radical, an amino radical, a (di)alkyl(C₁-C₂)amino radical;
- a phenyl radical optionally substituted by one or more radicals selected from a C₁-C₄ alkyl radical, a C₁-C₄ hydroxyalkyl radical.

3. Composition according to Claim 1, in which the radicals R₁ and R₂ form, together with the nitrogen atoms to which they are attached, a saturated or unsaturated 5- or 6-membered ring which is optionally substituted.

4. Composition according to any one of the preceding claims, in which the compounds (I) or (II) are such that Z represents a radical NR₃R₄.

5. Composition according to Claim 1, in which Z represents a radical NR₃R₄ and the radicals R₃ and R₄ form, together with the nitrogen atom to which they are attached, a ring of 5 to 7 members which is selected from the heterocycles pyrrolidine, piperidine, homopiperidine, piperazine, homopiperazine, morpholine; it being possible for said heterocycles to be substituted by one or more radicals selected from a hydroxyl, amino, (di)alkyl(C₁-C₂) amino, (di)hydroxyalkyl(C₁-C₂) amino, (di)alkyl(C₁-C₂) carboxamido, carboxyl, C₁-C₄ alkyl radical optionally substituted by one or more radicals selected from a hydroxyl, amino, C₁-C₂ (di)alkylamino radical.

6. Composition according to any one of the preceding claims, in which U represents CR or N, and R represents a hydrogen atom, a methyl radical, a methoxy radical, a 2-hydroxyethoxy radical, a methylamino radical, a dimethylamino or hydroxyethylamino radical or a dihydroxyethylamino radical or a methyl(hydroxyethyl)-amino radical.

7. Composition according to any one of the preceding claims, in which X represents a hydroxyl radical; a group NR'₁R"₁ with R'₁ and R"₁ selected independently from a hydrogen atom; a C₁-C₆ alkylradical optionally substituted by one or more hydroxyl radicals.

8. Composition according to any one of the preceding claims, in which X represents a group NR'₁R"₁ with R'₁ and R"₁ forming a heterocycle selected from the heterocycles pyrrolidine, piperidine, homopiperidine, piperazine, homopiperazine, morpholine; it being possible for said heterocycles to be substituted by one or more radicals selected from a halogen atom, an amino, (di)alkyl(C₁-C₄)amino, hydroxyl, carboxyl, (di)alkylcarboxamido, (C₁-C₂)alkoxy, C₁-C₄ alkyl radical which are optionally substituted by one or more radicals selected from a hydroxyl, amino, (di)alkylamino, alkoxy, carboxyl, sulfonyl radical.

9. Composition according to any one of the preceding claims, in which the compounds of formula (I) and (II) are such that V represents an oxygen atom, a group NR'₁ in which R'₁ represents a hydrogen atom or a C₁-C₆ alkyl radical optionally substituted by one or more hydroxyl radicals, a group N+R'₁R"₁ in which R'₁ and R"₁ are selected independently from a hydrogen atom, a C₁-C₆ alkyl radical optionally substituted by one or more hydroxyl radicals, or in which R'₁ and R"₁ may form, together with the nitrogen atom to which they are attached, a saturated or unsaturated heterocycle containing 5 to 7 members.

10. Composition according to any one of the preceding claims, in which Y, which are identical or different, represent a hydroxyl radical; a C₁-C₄ alkyl radical; a halogen atom; an oxygen atom substituted by a C₁-C₄ alkyl radical that may be substituted by one or more hydroxyl radicals; a group NR'₂R'₃; R'₂ and R'₃, which are identical or different, may be selected from a hydrogen atom; a C₁-C₄ alkylcarbonyl radical optionally substituted by a quaternary ammonium group or R'₂ and R'₃ may form, together with the nitrogen atom to which they are attached, a saturated heterocycle containing 5 to 7 members.

11. Composition according to any one of the preceding claims, **characterized in that** it further comprises an oxidant.

12. Compounds selected from the leuco compounds of formula (I), the azomethine dyes with a pyrazolinone unit of formula (II) corresponding to the compounds of formula (I) as defined in any one of Claims 1 to 10, their mesomeric forms, and also their acid addition salts and their solvates.

13. Method of dyeing keratin fibres, **characterized in that** at least one dyeing composition comprising at least one compound selected from the leuco compounds of formula (I) is applied to these fibres, the azomethine dyes with a pyrazolinone unit of formula (II) corresponding to the compounds of formula (I) as defined in any one of Claims 1 to 10, their mesomeric forms, and also their acid addition salts and their solvates.

14. Multi-compartment device, or multi-compartment dyeing kit, of which a first compartment contains at least one compound of formula (I), as defined in any one of Claims 1 to 10, and a second compartment contains an oxidant, and optionally a compound of formula (II), as defined in any one of Claims 1 to 10, an alkaline agent.
